# EUROPEAN PATENT APPLICATION

(11) **EP 4 001 280 A1**
(43) Date of publication of application: **25.05.2022**
(21) Application number: 20842975.3
(22) Date of filing: 17.07.2020
(51) Int. Cl.: C07D 471/08

(54) **METHOD FOR PRODUCING PHENOL DERIVATIVE**

(30) Priority: 19.07.2019 JP 2019134083
(71) Applicant: Nippon Chemiphar Co., Ltd., Tokyo 101-0032 (JP)
(72) Inventor: HIROSE Masaaki, Misato-shi, Saitama 341-0005 (JP)
(74) Representative: Godemeyer Blum Lenze Patentanwälte Partnerschaft mbB - werkpatent
(86) International application number: PCT/JP2020/027769
(87) International publication number: WO 2021/015109

(57) **Abstract**

Provided is a method for producing a phenol derivative represented by General Formula (B) and the like, including the step of: allowing metal lithium and liquid ammonia or a primary amine to act on a compound represented by General Formula (A) and the like: 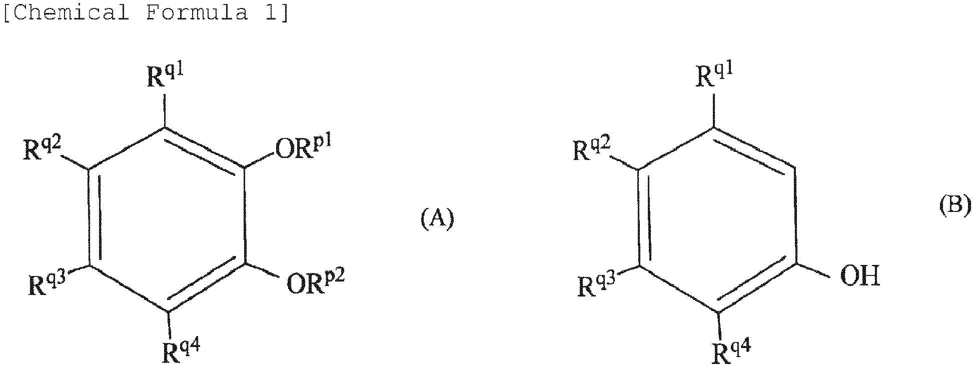 wherein R^{p1} represents a C₆₋₁₀ aryl group optionally having a substituent, R^{p2} represents a lower alkyl group and the like, and R^{q1}, R^{q2}, R^{q3}, and R^{q4} represent a hydrogen atom or any substituent, or adjacent two of R^{q1}, R^{q2}, R^{q3}, and R^{q4} optionally form a ring.

## Description

### Technical Field

The present invention relates to a method for producing a phenol derivative.

The present application claims priority based on Patent Application No. 2019-134083 filed in Japan on July 19, 2019, the contents of which are incorporated herein by reference.

### Background Art

Among the compounds having a morphinan skeleton represented by the following formula, compounds that bind to an opioid receptor and exhibit excellent pharmacological activity such as analgesia and antitussive effect, like morphine and buprenorphine, are known.

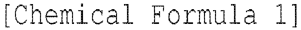

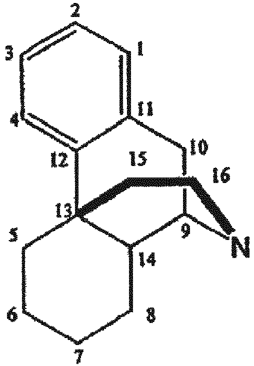

(In the present specification, when only the position number is described, it indicates the position number of the structural formula.)

Though among these compounds, a number of compounds having an ether bridge (commonly called 4,5-epoxy ring) between the 4-position and the 5-position of the morphinan skeleton are known, as semi-synthetic pharmaceuticals, many derivatives having no ether bridge between the 4-position and the 5-position of the morphinan skeleton, like butorphanol and levallorphan, have also been reported. 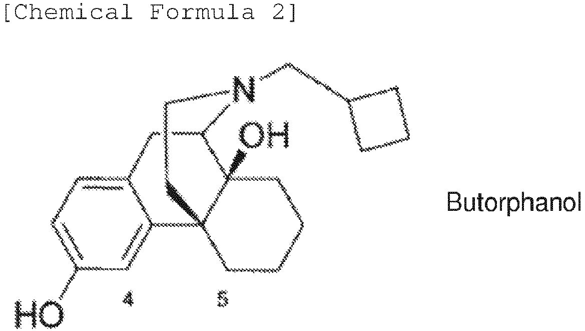 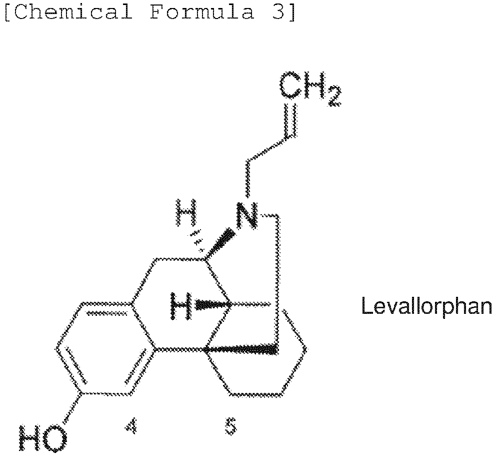 In the production of these derivatives, as described below, a technique is widely used in which a known morphinan derivative (i) having a 4,5-epoxy ring in a morphinan skeleton is used as a starting material, the 4,5-epoxy ring is opened to form a 4-position phenol form (ii) in the first step, then the phenolic hydroxyl group at the 4-position produced is converted to a phenoxy group by the Ullmann reaction or the like to form a compound (iii)-1 in the second step, and the phenoxy group at the 4-position is removed by the Birch reduction, the Benkeser reduction or the like in which an alkali metal is used in the third step to obtain a compound (iv) (route 1). As another method, a method has also been reported in which the phenolic hydroxyl group produced in the first step is triflated in the second step to form a compound (iii)-2, and then a trifluoromethylsulfonyloxy group is removed by a palladium-catalyzed reaction in the presence of a nucleophile that serves as a hydride source such as triethylsilane in the third step to obtain a compound (iv) (route 2). 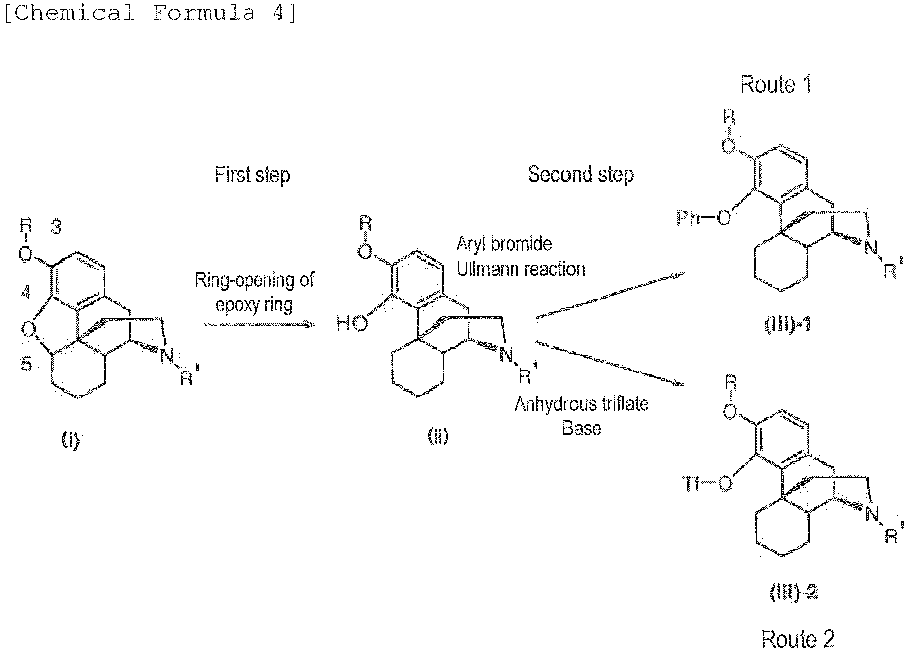 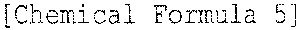 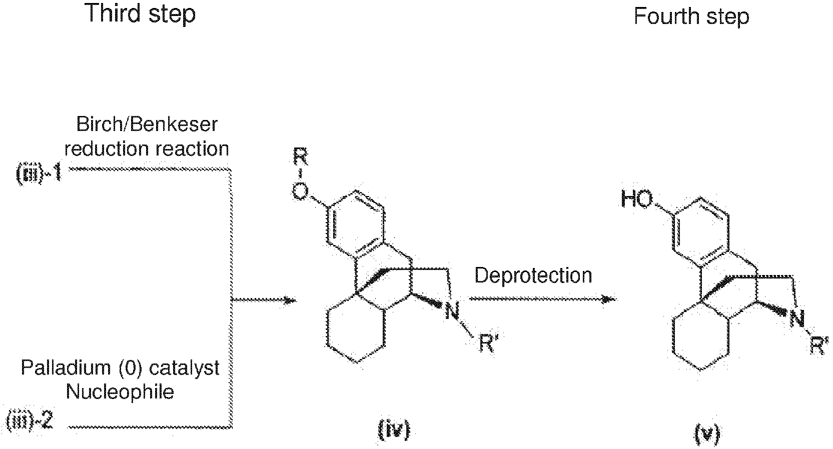 These reactions are usually performed in many cases using a derivative in which the phenolic hydroxyl group at the 3-position which plays an important role in the expression of biological activity is protected with an alkyl group (mainly a methyl group) or the like. Thus, in the production of the physiologically active substance (v) as a final target, a step of removing an alkyl group introduced as a protecting group of the hydroxyl group at the 3-position is separately required finally, and removal of, in particular, a methyl group has many problems in application to industrial scale production such as use of a reagent having high toxicity and corrosiveness such as boron tribromide.

In the case where the dehydroxylation reaction in the third step is performed by the Birch reduction or the Benkeser reduction, many examples in which sodium is used as an alkali metal have been reported.

For example, Non-Patent Literature 1 discloses a synthesis example of a compound 2 that is dephenoxylated by allowing metal sodium to act on a compound 1 having a 2,4-diaminophenyloxy group on a benzene ring in liquid ammonia. 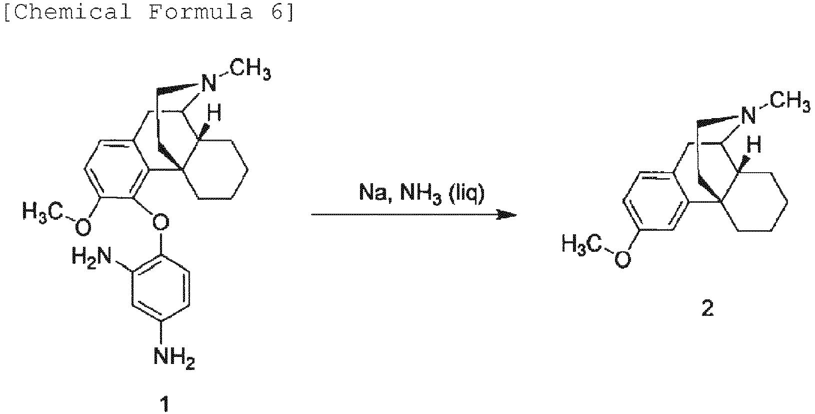 EXAMPLE 1 of Patent Literature 1 discloses an example of synthesis of 14β-hydroxy-3-methoxymorphinan (IV) by allowing metal sodium to act on 4-phenoxy-14β-hydroxy-3-methoxymorphinan (III) at -40°C in liquid ammonia. 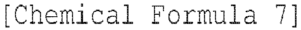 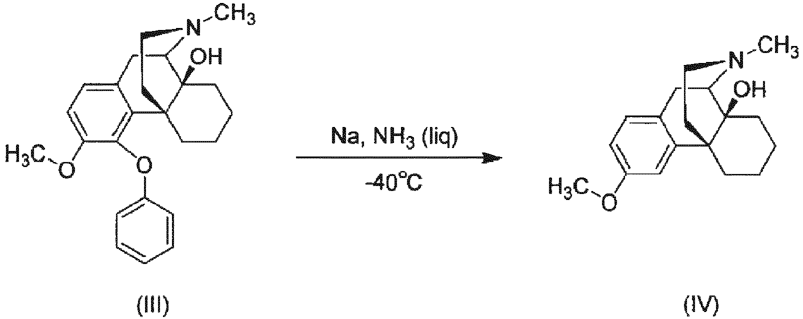 Non-Patent Literature 2 discloses a synthesis example of a compound 12 that is dephenoxylated by allowing metal sodium to act on a compound 11 having a phenoxy group at -78°C to room temperature in liquid ammonia. 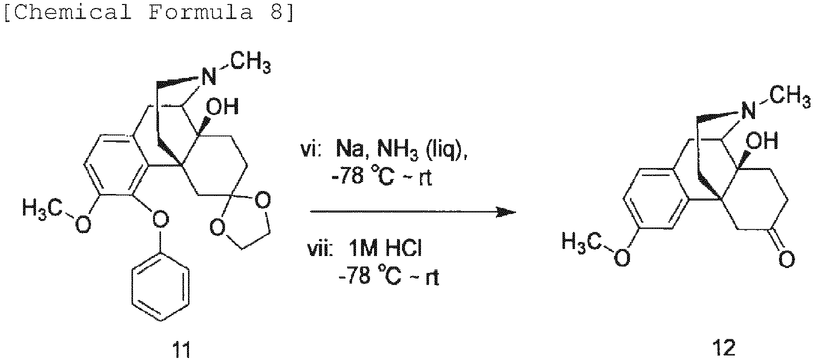 Non-Patent Literature 3 also describes the same dephenoxylation reaction as in Non-Patent Literature 2. That is, Scheme 1 discloses a method in which a compound 11 is subjected to a conversion reaction in 3 steps to obtain a compound 12a, which is further changed to a compound 13a, and then a demethylation reaction is performed by allowing boron tribromide to act on the compound in methylene chloride to obtain a compound 6a. 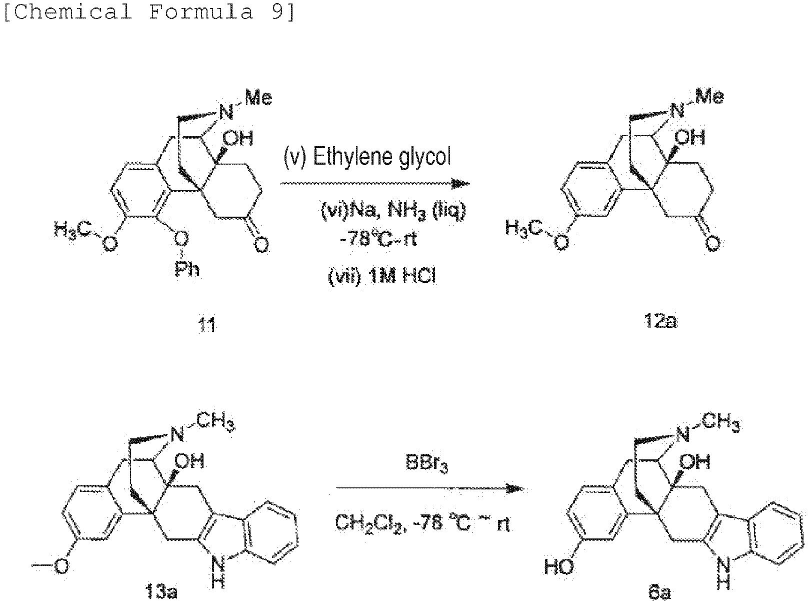 Scheme 1 of Non-Patent Literature 4 discloses a synthesis example of a compound 16 that is dephenoxylated by reacting metal sodium with a compound 15 at -78°C in the presence of liquid ammonia. 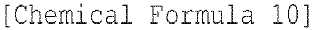 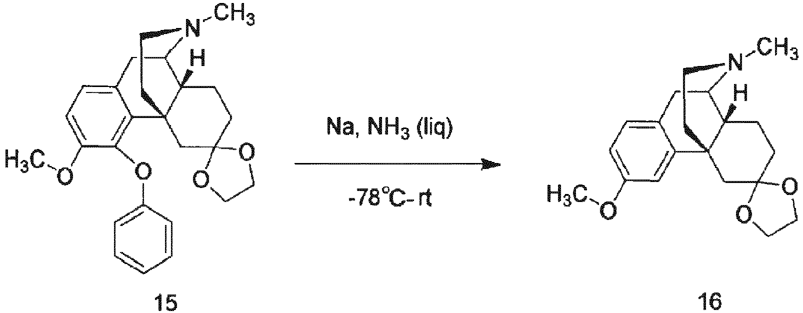 Further, Scheme 2 of Non-Patent Literature 4 discloses an example in which a compound 21a is obtained from the compound 16 through several steps, and then demethylation reaction is performed using boron tribromide to obtain a compound 22a. 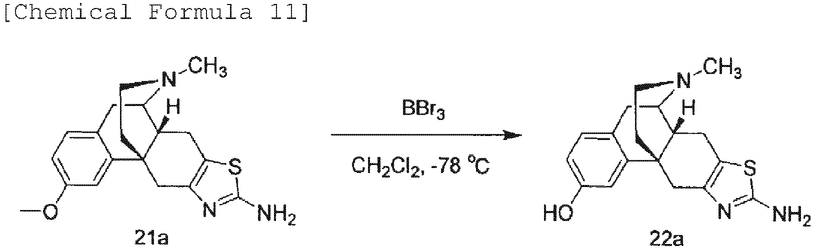 Non-Patent Literature 5 discloses an example in which metal sodium is allowed to act on a compound (XVIII) in which the phenolic hydroxyl group at the 4-position has been converted to a phenoxy group at -55°C in toluene in the presence of liquid ammonia in the same manner as in Patent Literature 1 to perform a dephenoxylation reaction, thereby a compound (XIX) is obtained. 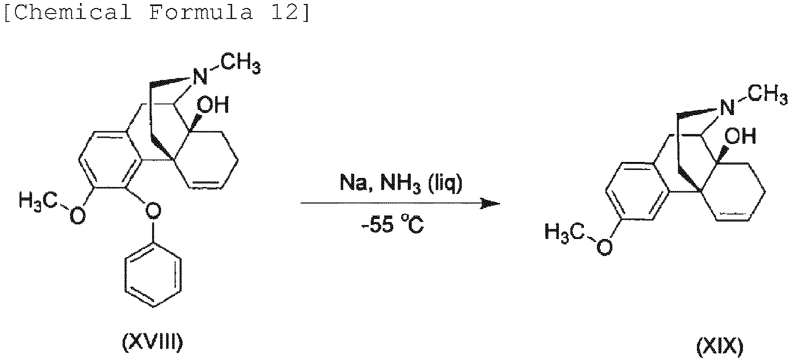 Non-Patent Literature 6 discloses a production method of (+)-deoxynaltrexone in which 4,5-epoxy from (+)-naltrexone is ring-opened. In the Literature, a method is disclosed in which metal sodium is allowed to act on a compound 23 in liquid ammonia to obtain a compound 24 from which a phenoxy group is removed. Further, the obtained compound 24 is treated with 2 N hydrochloric acid to remove the acetal protecting group, thereby the compound 24 is changed to a compound 25. Then a demethylation reaction with boron tribromide is performed to obtain (+)-deoxynaltrexone 4. 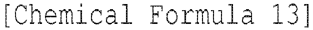 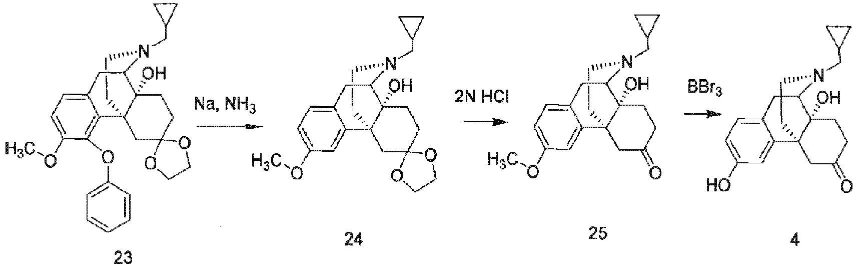 In Non-Patent Literature 7, liquid ammonia and metal sodium are allowed to act on a compound 10 having a buprenorphine skeleton in diethyl ether to obtain a compound 11 that is dephenoxylated is obtained. 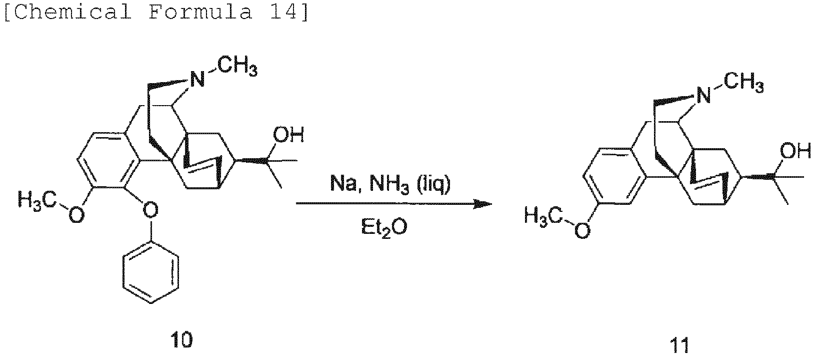 Non-Patent Literature 8 discloses a method in which a compound 7d is changed to a ketal protecting form according to a conventional method and then subjected to the Birch reduction with metal sodium in toluene for dephenoxylation, then the ketal protecting group is removed, and a pyridine hydrochloride is allowed to act on the obtained a compound 8d at 170°C to perform a 3-position demethylation reaction, thereby a compound 9d is obtained. 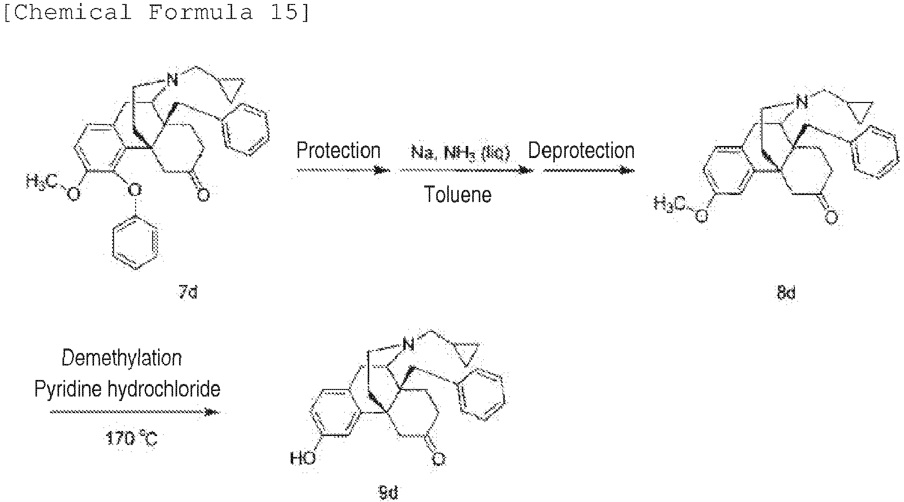 Supplementary material of Non-Patent Literature 9 describes a method in which metal sodium is allowed to act on a compound S3 in the presence of liquid ammonia in toluene to change the compound S3 to a compound 1a.

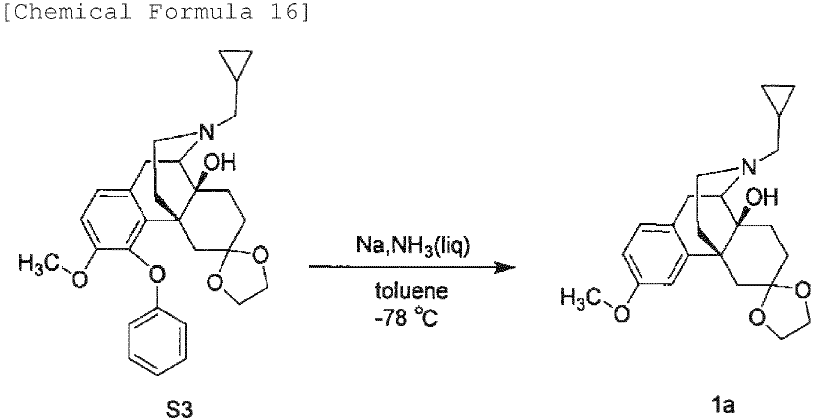

Though in these production methods, metal sodium is used for the dephenoxylation reaction, metal sodium has very high reactivity, and easily reacts with slight moisture to generate hydrogen, and there is a risk that hydrogen is ignited by heat of reaction at that time and further explodes. Low temperature conditions such as -40°C and -78°C are required as reaction conditions, and when a severe exothermic reaction occurs with scale-up, control of the temperature becomes difficult. Thus, it is difficult to apply these production methods to industrial scale production. For these reasons, development of a dephenoxylation reaction by a safer reagent or technique instead of metal sodium is desired.

In order to avoid these risks, a reagent in which metal sodium is supported on a solid phase has been developed, and an example of performing a reaction for removing the phenoxy group at the 4-position of a morphinan skeleton using this reagent has also been reported.

For example, Scheme 1 of Non-Patent Literature 10 discloses a method in which metal sodium supported on silica gel is allowed to act on a compound 5 under a Benkeser reduction condition in THF to form a compound 6, and then, after further several subsequent functional group conversions, boron tribromide is allowed to act on the obtained compound 8a to perform a demethylation reaction of the hydroxyl group at the 3-position to obtain a compound 2a. 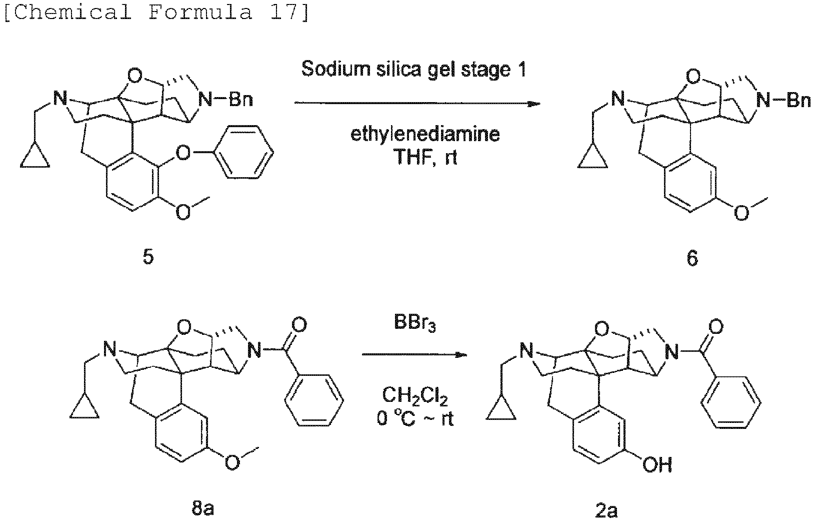 Non-Patent Literature 11 discloses an example (Scheme 2) in which metal sodium supported on silica gel is allowed to act on a compound 6 in the same manner as in Non-Patent Literature 10 to form a compound 7a (Scheme 1), and then a demethylation reaction is performed using boron tribromide to obtain a compound 3a.

For the same conversion reaction, paragraph numbers [0038] and [0039] (Reference Example 3) of Patent Literature 2 describe a production method of the compound 7a in which metal sodium supported on silica gel is used. 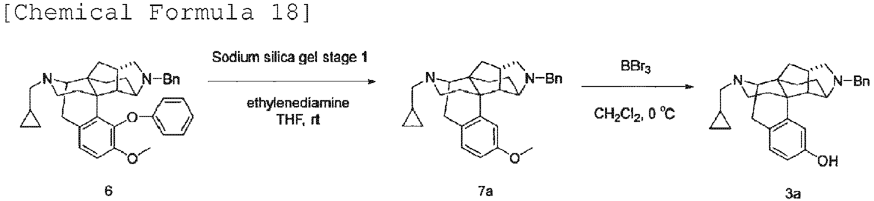 Further, paragraph number [0025] of Patent Literature 3 discloses a method in which the Ullmann reaction is performed on a compound (b-1) to form a compound (m), then metal sodium supported on silica gel is allowed to act on the compound under a Benkeser reduction condition to perform a dephenoxylation reaction, thereby the compound is changed to a compound (n), and a demethylation reaction with boron tribromide or the like is further performed to obtain a compound (o). Paragraph number [0024] of Patent Literature 4 and paragraph number

of Patent Literature 5 also disclose a production method of a compound (n) by a Benkeser reduction reaction in which the same metal sodium supported on silica gel is used. 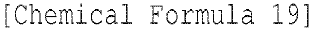 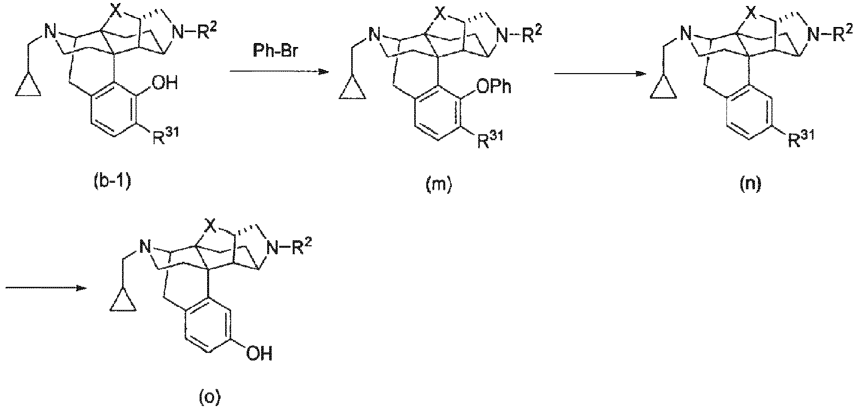 Though these methods for removing the hydroxyl group at the 4-position using metal sodium supported on silica gel are useful for synthesis on a laboratory scale in that the risk of ignition is low and the reaction can be performed easily as compared with the method reported so far in which metal sodium is used, use of the present reagent for production on an industrial scale is limited because metal sodium supported on silica gel is an expensive reagent and is difficult to obtain in large quantities.

In order to solve these problems, a method of dehydroxylating a morphinan skeleton 4-position triflate form by a palladium-catalyzed reaction has been recently reported.

For example, Non-Patent Literature 12 reports an example in which a catalytic amount of palladium acetate and 1,3-bis(diphenylphosphino)propane are allowed to act on a compound 15 in the presence of triethylsilane to perform a dehydroxylation reaction, thereby a compound 18 is obtained. 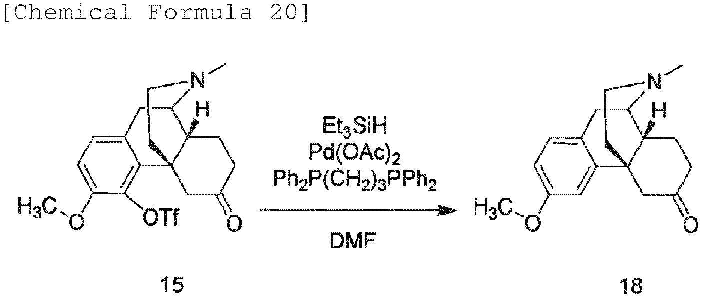 Paragraph number [0514] of Patent Literature 6 shows an example in which a compound 6 is subjected to dehydroxylation under the same reaction conditions as in Non-Patent Literature 12 described above in THF to obtain a compound 7.

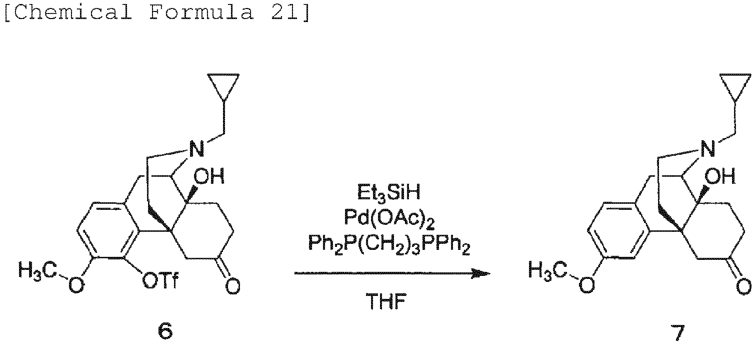

For the same conversion reaction, paragraph numbers [0287] and [0288] of Patent Literature 7 disclose an example in which DMF is used as a solvent.

Further, paragraph numbers [0393] and [0394] of Patent Literature 8 disclose an example in which the hydroxyl group at the 4-position of a compound 1 is removed under the same reaction conditions as in Patent Literature 6 in DMF to form a compound 2, and then an ethanethiol sodium salt is allowed to act on the compound 2 at 150°C in NMP to perform demethylation, thereby a compound 3 is obtained. 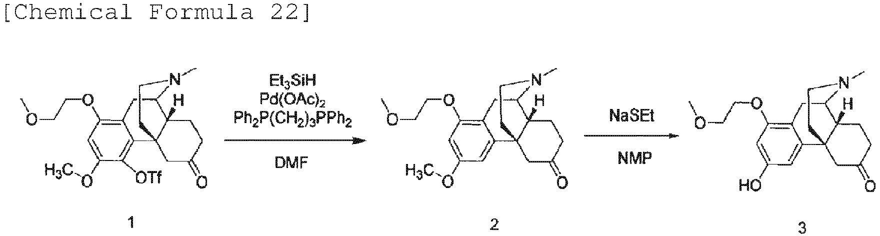 Paragraph numbers [0160] to [0165] of Patent Literature 9 describe an example in which the hydroxyl group at the 4-position of the compound 1 is removed under the same reaction conditions as in Patent Literature 6 and the like in DMF, and then ketal protection of a ketone moiety of the compound 2 and removal of a benzyl group as a protecting group of the hydroxyl group at the 3-position are performed to change the compound 2 to the compound 3. 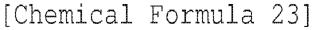 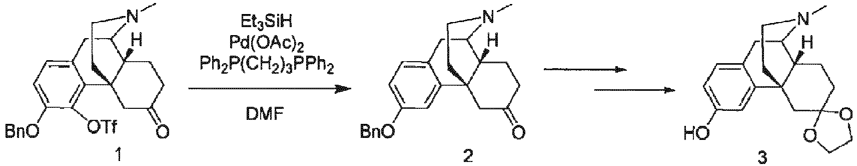 Example 10 of Patent Literature 10 reports an example in which the hydroxyl group at the 4-position of a compound AE is removed under the same reaction conditions as in Patent Literature 6 and the like in DMF to obtain a compound AF. 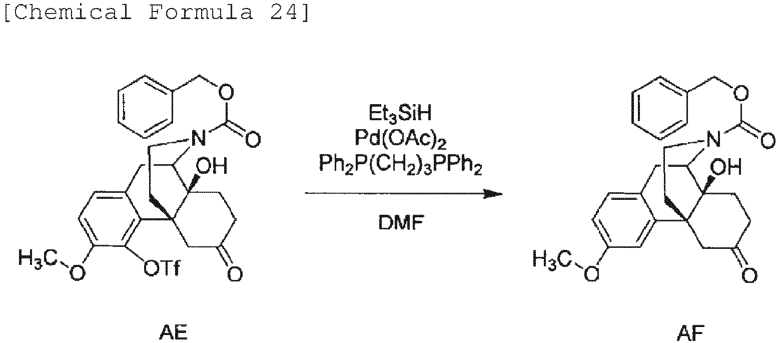 As an example of removing the hydroxyl group at the 4-position without using triflate, Non-Patent Literature 13 reports a method in which the phenolic hydroxyl group at the 4-position of a compound 27 in which a 1-phenyl-tetrazolyl-5-yl group is introduced into the hydroxyl group at the 4-position is removed by a catalytic hydrogenation reaction in the presence of a palladium carbon catalyst to obtain a compound 5. 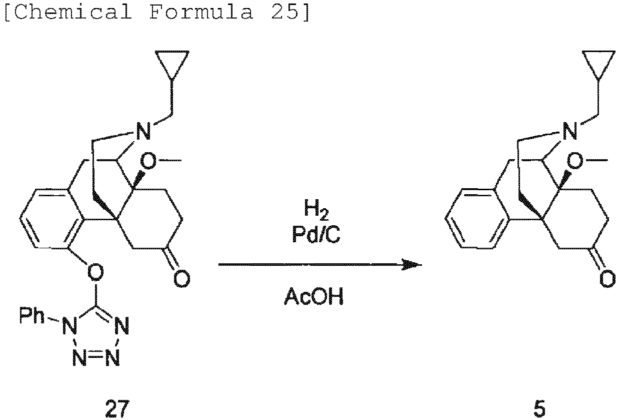 Non-Patent Literature 14 also reports a similar method for removing the hydroxyl group at the 4-position as a method for obtaining a compound 21 from a compound 28. 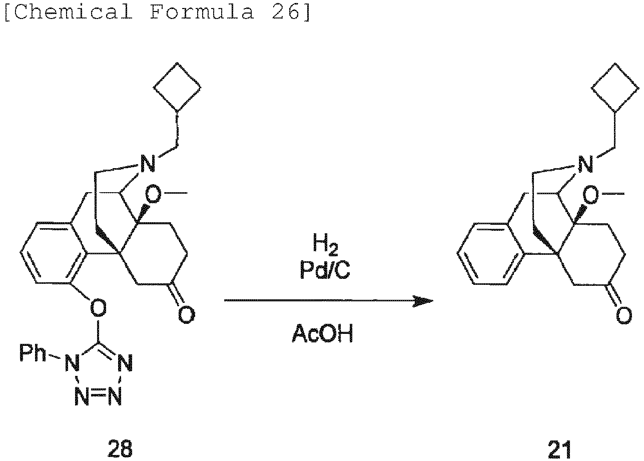 Though both of the method of converting the hydroxyl group at the 4-position into triflate and then removing it by a palladium-catalyzed reaction and the method of converting the hydroxyl group at the 4-position to a 1-phenyl-1H-tetrazolyl-5-yloxy group and then removing it by a catalytic hydrogenation reaction in the presence of a palladium carbon catalyst are excellent in that metal sodium, which react vigorously with water and has a risk of ignition and explosion, is not used, the introduction is sometimes difficult, further, there remains a problem that the palladium-catalyzed reaction, which is a removal reaction after the introduction, does not proceed in a derivative having a large steric hindrance and the like, and there are few application examples at present.

### Citation List

### Patent Literature

Patent Literature 1: US 5504208
Patent Literature 2: JP 2015-180605 A
Patent Literature 3: WO 2013/035833
Patent Literature 4: WO 2014/136305
Patent Literature 5: WO 2014/021273
Patent Literature 6: WO 2014/102593
Patent Literature 7: WO 2014/102587
Patent Literature 8: WO 2016/182840
Patent Literature 9: US 2015/0072971 A1
Patent Literature 10: WO 2013/167963
Patent Literature 11: WO 2016/148232

### Non-Patent Literature

Non-Patent Literature 1: Journal of Organic Chemistry, 30 (6), 1769-1773, 1965
Non-Patent Literature 2: Bioorganic & Medicinal Chemistry Letters, 19 (16), 4603-4606, 2009
Non-Patent Literature 3: Chemical Biology & Drug Design, 74 (4), 335-342, 2009
Non-Patent Literature 4: Journal of Medicinal Chemistry, 50 (11), 2747-2751, 2007
Non-Patent Literature 5: Tetrahedron, 24 (20), 6185-96, 1968
Non-Patent Literature 6: Journal of Medicinal Chemistry, 58 (12), 5038-5052, 2015
Non-Patent Literature 7: Recueil des Travaux Chimiques des Pays-Bas, 107 (6), 449-54, 1988
Non-Patent Literature 8: Helvetica Chimica Acta, 73 (2), 326-36, 1990
Non-Patent Literature 9: Journal of Organic Chemistry, 73, 8093-8096, 2008
Non-Patent Literature 10: Bioorganic & Medicinal Chemistry Letters, 27 (12), 2742-2745, 2017
Non-Patent Literature 11: Bioorganic & Medicinal Chemistry Letters, 27 (15), 3495-3498, 2017
Non-Patent Literature 12: Tetrahedron Letters, 51 (17), 2359-2361, 2010
Non-Patent Literature 13: Journal of Medicinal Chemistry, 33 (4), 1200-1206, 1990
Non-Patent Literature 14: Helvetica Chimica Acta, 72 (6), 1233-1240, 1989
Non-Patent Literature 15: Bioorganic & Medicinal Chemistry Letters, 22, 7711-7714, 2012
Non-Patent Literature 16: Bioorganic & Medicinal Chemistry Letters, 22, 5071-5074, 2012

### Summary of Invention

### Technical Problem

Under the above circumstances, the method for removing the hydroxyl group at the 4-position using the Birch reduction or the Benkeser reduction is based on an electron transfer reaction, is hardly affected by steric hindrance, thus can be applied to the production of derivatives having a wide range of structures, and is an attractive method for removing the hydroxyl group at the 4-position, which is still widely used, though severe reactivity of metal sodium with water remains as a problem.

Thus, the present inventors have devised to use metal lithium having relatively low reactivity with water for the reaction for removing the hydroxyl group at the 4-position, instead of metal sodium that vigorously reacts with water.

Though metal lithium reacts with water at room temperature, the reaction is relatively mild. Thus, the risk is lower than that of metal sodium, and metal lithium can be said to be an alkali metal that can be easily used even on an industrial scale. In fact, metal sodium needs to be stored under water-free conditions with being immersed in mineral oil or the like, whereas metal lithium can be stored without being immersed in mineral oil or the like and without blocking air and moisture in a brown bottle or the like, similarly to normal reagents.

In addition, an operation of processing a block of metal sodium into small pieces is necessary to dissolve metal sodium in a solvent such as liquid ammonia before the reaction under the conditions of the Birch reduction, and the industrial use is difficult from the viewpoint of risks, labor and the like at the time of processing. As one means for compensating for these problems, fine granular metal sodium dispersed in mineral oil is also commercially available. However, it is expensive, and in addition, in recent years, some manufacturers stopped the supply from the viewpoint of low demand and reagent safety, and its availability may not be continuous. Meanwhile, metal lithium is relatively stable, and thus metal lithium processed into a granular shape can be easily obtained at low cost, and it can be used on an industrial scale without pretreatment, which can be a great advantage.

Thus, if the Birch reduction or the Benkeser reduction for dehydroxylating the 4-position of a morphinan skeleton can be performed with metal lithium instead of metal sodium, a larger number of morphinan derivatives in which the 4-position is dehydroxylated can be produced on an industrial scale.

However, there has been no case of using metal lithium in the Birch reduction or the Benkeser reduction for the purpose of removing the phenolic hydroxyl group at the 4-position of the morphinan skeleton.

Though two cases have been reported in Non-Patent Literature 15 and Non-Patent Literature 16 as examples in which Birch reduction or Benkeser reduction in which metal lithium is used is applied to synthesis of analogs having a morphinan skeleton, but both are not for the purpose of removing the phenolic hydroxyl group at the 4-position.

The example of Non-Patent Literature 15 reports that a compound 6 in which the benzene ring of the morphinan skeleton has been reduced and a compound 7 in which the reduction has further progressed were obtained by allowing metal lithium to act on a compound 5 in the presence of liquid ammonia at -33°C in ethanol-THF. Non-Patent Literature 6 also describes reactions on the same compounds. 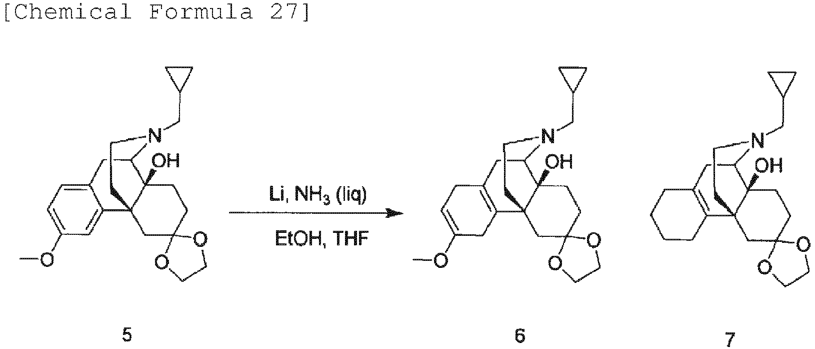 These reports suggest that "In the Birch reduction or the Benkeser reduction in which metal lithium is used on a derivative having a morphinan skeleton, an undesirable reduction reaction of a benzene ring proceeds to produce a complicated by-product, and thus control of the reaction for the purpose of producing the 4-position dehydrated form shown in the scheme of paragraph number [0006] of the present specification is difficult.".

Presumably because of this, metal sodium has been used instead of metal lithium in the Birch reduction or the Benkeser reduction for the purpose of removing the phenolic hydroxyl group at the 4-position of the morphinan skeleton derivative.

Under such circumstances, as a result of intensive studies, the present inventors have successfully found a condition under which the desired removal reaction of the phenolic hydroxyl group at the 4-position proceeds in high yield while controlling the reduction reaction of a benzene ring of a morphinan skeleton when metal lithium is allowed to act on a specific morphinan derivative represented by General Formula (I) below in the presence of an amine.

Surprisingly, the present inventors have also found in the present reaction, a methyl group which is a protecting group of the phenolic hydroxyl group at the 3-position is also removed simultaneously with dehydroxylation at the 4-position.

This represents that "In the method by the 4-position dehydroxylation reaction by the Birch reduction or the Benkeser reduction in which metal lithium is used, two functional group conversions of 4-position dehydroxylation and 3-position demethylation can be achieved in one step.", which can greatly shorten the industrialization process, in contrast to "In the conventional method by a 4-position dehydroxylation reaction by the Birch reduction or the Benkeser reduction with metal sodium, in the production of a derivative having a 3-position phenolic hydroxyl group, two steps of (1) a 4-position dehydroxylation step and (2) a 3-position hydroxyl group deprotection step (demethylation when the protecting group is a methyl group) are required.".

Further, it has been found that in the 4-position dehydroxylation reaction by the Birch reduction or the Benkeser reduction in which metal lithium is used in the present invention, the debenzylation reaction also proceeds simultaneously, while the benzyl group as a protecting group on a nitrogen atom is unreacted, and the benzyl group needs to be separately removed by a catalytic hydrogenation reaction or the like in the presence of a palladium carbon catalyst, for example, in paragraph number [0025] [Chemical Formula 7] of Patent Literature 3 among the examples of the 4-position dehydroxylation reaction by the Birch reduction or the Benkeser reduction in which metal sodium is used in Patent Literatures 2 to 5 and Non-Patent Literatures 10 and 11. For example, to obtain (1S,3aR,5aS,6R,11bR,11cS)-14-(cyclopropylmethyl)-2,3,3a,4,5,6,7,11c-octahydro-1H-6,11b-(epiminoethano)-1,5a-methanonaphtho[1,2-e]indole-10-ol (compound A) described in Patent Literature 11, which is an important synthetic intermediate of a morphinan derivative having an excellent opioid δ receptor agonist action, for example, in Patent Literature 3, sodium supported on silica gel is allowed to act on a compound 74 under the Benkeser reduction conditions described in paragraph numbers [0283] to [0285] (Example 65) to perform a 4-position dehydroxylation reaction to form a compound 75, then, debenzylation by hydrogenation reaction described in paragraphs [0289] to [0291] (Example 67) is performed to form a compound 76, and then a demethylation reaction in which boron tribromide is used described in paragraph numbers [0023] to [0025] (Reference Example 1-1) of Patent Literature 11 is performed: in total three steps are necessary.

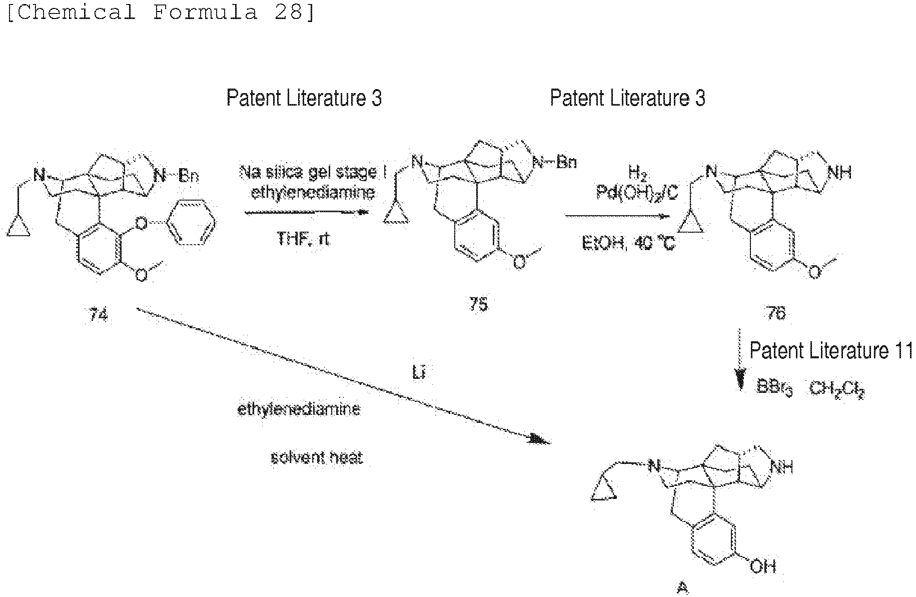

As a result of intensive studies, the present inventors have found that the intended dephenoxylation reaction at the 4-position proceeds under the conditions of the Birch reduction or the Benkeser reduction in which metal lithium is used. Further, the present inventors have found that when a methoxy group and an N-benzyl group are present at the 3-position as in a compound 74 of Example 64 of Patent Literature 3, demethylation and further N-debenzylation proceed at once, and three functional group conversions can be conveniently performed by One-Pot, thereby completing the present invention.

One object of the present invention is to provide a production method of a monophenol derivative, a method of converting a biphenyl ether form having an ortho-methoxy group into a monophenol derivative in one step. The method provided by the present invention can be applied to a compound having a morphinan skeleton. As one embodiment, the present invention provides a production method of a highly versatile morphinan derivative that is applicable to a dephenoxylation reaction of a morphinan derivative having a phenoxy group at the 4-position.

### Solution to Problem

[1] That is, one embodiment of the present invention relates to a method for producing a phenol derivative represented by General Formula (B) below: 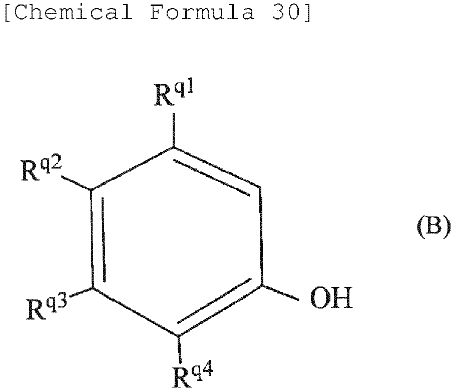
   wherein R^{q1}, R^{q2}, R^{q3}, and R^{q4} are same or different, and represent a hydrogen atom or any substituent, or
   adjacent two of R^{q1}, R^{q2}, R^{q3}, and R^{q4} optionally form a ring; or
   a salt, a tautomer, a stereoisomer, or an isotope of the phenol derivative, comprising the step of:
      allowing metal lithium and liquid ammonia or a primary amine to act on a compound represented by General Formula (A) below: 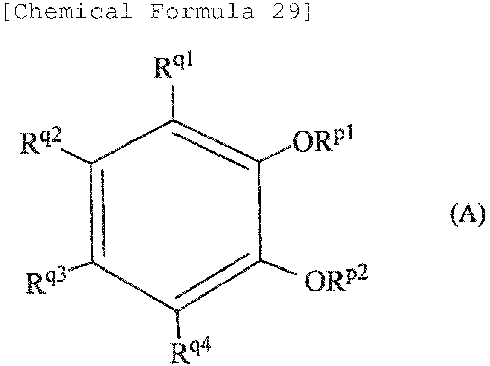
      wherein R^{p1} represents a C₆₋₁₀ aryl group optionally having a substituent,
      R^{p2} represents a lower alkyl group, an aralkyl group optionally having a substituent, an alkenyl group optionally having a substituent, or a silyl protecting group, and
      R^{q1}, R^{q2}, R^{q3}, and R^{q4} have a meaning same as above; or
      a tautomer, a stereoisomer, or an isotope of the compound.
[2] One embodiment of the present invention relates to the method according to [1] above, wherein R^{q3} and R^{q4} are a hydrogen atom, and R^{q1} and R^{q2} together form a ring.
[3] One embodiment of the present invention relates to the method according to [1] or [2] above, wherein R^{p1} is a phenyl group.
[4] One embodiment of the present invention relates to the method according to any one of [1] to [3] above, wherein RP2 is a methyl group.
[5] One embodiment of the present invention relates to a method for producing a morphinan derivative represented by General Formula (II) below: 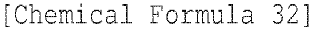 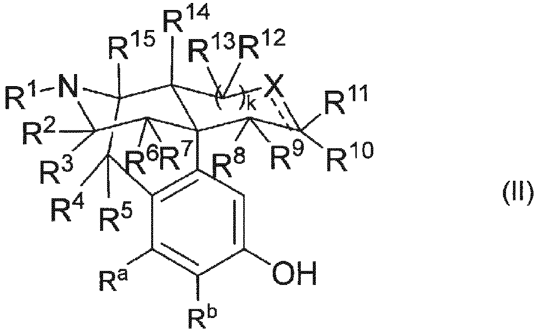
   wherein R¹ to R¹⁵, R^{a}, and R^{b} are same or different, and represent a hydrogen atom or any substituent
   wherein any two groups selected from R⁸, R¹⁰, and R¹⁴ are optionally bonded to each other to form an alkylene chain, the alkylene chain is optionally substituted with a substituent, a carbon atom that constitutes the alkylene chain is optionally replaced with a heteroatom, the alkylene chain optionally further has a double bond or an amide bond in middle, and rest other than the selected two groups is optionally bonded to the alkylene chain,
   X indicates a carbon atom optionally having a substituent, a nitrogen atom optionally having a substituent, or an oxygen atom,
   k represents 1 or 2, and
   a double line consisting of a solid line and a broken line represents a single bond or a double bond wherein
   when the double line consisting of a solid line and a broken line is a double bond,
   X is N or CR⁰¹ wherein R⁰¹ is a hydrogen atom or any substituent, and in this case R¹¹ is absent,
   when the double line consisting of a solid line and a broken line is a single bond,
   X is O, NR⁰², CR⁰³R⁰⁴, C=CR^{c0}R^{d0}, or C=NCₓ
   wherein R⁰², R⁰³, and R⁰⁴ are same or different, and are a hydrogen atom or any substituent,
   R^{c0} and R^{d0} are same or different, and are a hydrogen atom, a C₁₋₁₀ alkyl group optionally having a substituent, a C₃₋₁₀ alkenyl group optionally having a substituent, an aryl group optionally having a substituent, or a heteroaryl group optionally having a substituent,
   Cₓ represents a C₁₋₁₀ alkyl group optionally having a substituent, and
   R¹⁰ and R¹¹, together with a carbon atom to which R¹⁰ and R¹¹ are bonded, represent C=CR^{c}R^{d} (R^{c} and R^{d} are same or different, and indicate a hydrogen atom, a C₁₋₁₀ alkyl group optionally having a substituent, a C₃₋₁₀ alkenyl group optionally having a substituent, an aryl group optionally having a substituent, or a heteroaryl group optionally having a substituent) or C=NC_{y} (C_{y} indicates a C₁₋₁₀ alkyl group optionally having a substituent), or optionally form a cyclic ketal optionally having a substituent, and further, when X is NR⁰², together with a carbon atom to which R¹⁰ and R¹¹ are bonded, are optionally a carbonyl group or a thiocarbonyl group,
   the R⁰¹ and R¹⁰ are optionally bonded to each other to form an unsaturated hydrocarbon ring optionally having a substituent, an unsaturated heterocycle optionally having a substituent, or a lactam ring optionally having a substituent, and the unsaturated hydrocarbon ring, the unsaturated heterocycle, and the lactam ring are optionally further fused with a saturated hydrocarbon ring optionally having a substituent, a saturated heterocycle optionally having a substituent, an unsaturated hydrocarbon ring optionally having a substituent, or an unsaturated heterocycle optionally having a substituent,
   the R⁰² or R⁰³ and R¹⁰ are optionally bonded to each other to form a saturated hydrocarbon ring optionally having a substituent, a saturated heterocycle optionally having a substituent, an unsaturated hydrocarbon ring optionally having a substituent, an unsaturated heterocycle optionally having a substituent, or a lactam ring optionally having a substituent, and the saturated hydrocarbon ring, the saturated heterocycle, the unsaturated hydrocarbon ring, the unsaturated heterocycle, and the lactam ring are optionally further fused with a saturated hydrocarbon ring optionally having a substituent, a saturated heterocycle optionally having a substituent, an unsaturated hydrocarbon ring optionally having a substituent, or an unsaturated heterocycle optionally having a substituent,
   the R^{c0} and R^{c}, together with C=C to which R^{c0} and R^{c} are bonded, optionally form an unsaturated hydrocarbon ring or an unsaturated heterocycle,
   the R^{c0} and C_{y}, together with C=C and C=N to which R^{c0} and C_{y} are bonded, optionally form an unsaturated heterocycle,
   the Cₓ and C_{y}, together with C=N to which Cₓ and C_{y} are bonded, optionally form an unsaturated heterocycle; or
   a salt, a tautomer, a stereoisomer, or an isotope of the morphinan derivative, comprising the step of:
      allowing metal lithium and an amine to act on a morphinan derivative represented by General Formula (I) below: 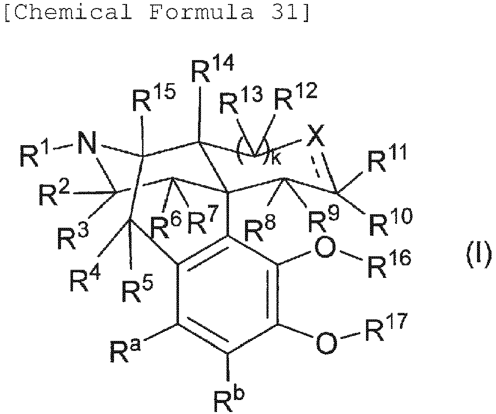
      wherein R¹ to R¹⁵, R^{a}, and R^{b} have a meaning same as above,
      R¹⁶ represents a C₆₋₁₀ aryl group optionally having a substituent or a heteroaryl group optionally having a substituent,
      R¹⁷ represents a C₁₋₁₀ alkyl group, an aralkyl group optionally having a substituent, an alkenyl group optionally having a substituent, or a silyl protecting group,
      X, a double line consisting of a solid line and a broken line, and k have a meaning same as above; or
      a tautomer, a stereoisomer, or an isotope of the morphinan derivative in presence or absence of an organic solvent.
[6] One embodiment of the present invention relates to the method according to [5] above, wherein k is 1, X is CH₂, and the double line consisting of a solid line and a broken line is a single bond.
[7] One embodiment of the present invention relates to the method according to [5] or [6] above, wherein R¹ is a hydrogen atom, a methyl group, a cyclopropylmethyl group, a cyclobutylmethyl group, a benzyl group, or an allyl group.
[8] One embodiment of the present invention relates to the method according to any one of [5] to [7], wherein R¹ is a hydrogen atom.
[9] One embodiment of the present invention relates to the method according to any one of [5] to [8] above, wherein R² to R⁷, R⁹, R¹¹ to R¹³, R¹⁵, R^{a}, and R^{b} are same or different, and are a hydrogen atom, a methyl group, a cyclopropylmethyl group, a cyclobutylmethyl group, a benzyl group, or an allyl group.
[10] One embodiment of the present invention relates to the method according to any one of [5] to [9] above, wherein R⁸, R¹⁰, and R¹⁴ together form two rings containing a carbon atom to which R⁸, R¹⁰, and R¹⁴ are bonded.
[11] One embodiment of the present invention relates to the method according to any one of [5] to [10] above, wherein R¹⁶ is a C₆₋₁₀ aryl group optionally having a substituent.
[12] One embodiment of the present invention relates to the method according to any one of [5] to [10] above, wherein R¹⁶ is a phenyl group.
[13] One embodiment of the present invention relates to the method according to any one of [5] to [12] above, wherein R¹⁷ is a methyl group.
[14] One embodiment of the present invention relates to the method according to any one of [5] to [13] above, wherein the amine is liquid ammonia, a primary amine, or a secondary amine.
[15] One embodiment of the present invention relates to the method according to any one of [5] to [13] above, wherein the amine is a primary amine.
[16] One embodiment of the present invention relates to the method according to any one of [5] to [13] above, wherein the amine is a primary amine represented by General Formula (III) below: 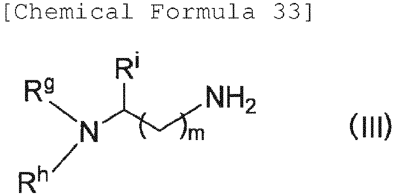 wherein R^{g}, R^{h}, and Rⁱ are same or different, and represent a hydrogen atom or a C₁₋₆ alkyl group optionally having a substituent or any two of R^{g}, R^{h}, and Rⁱ together optionally form a ring, and m represents an integer of 1 to 5.
[17] One embodiment of the present invention relates to the method according to any one of [5] to [16] above, wherein metal lithium is used in an amount of 2 to 20 equivalents relative to one functional group to be reduced in the morphinan derivative represented by the General Formula (I) .
[18] One embodiment of the present invention relates to the method according to any one of [5] to [17] above, wherein the organic solvent is an aromatic hydrocarbon solvent, an alcohol solvent, or an ether solvent.
[19] One embodiment of the present invention relates to the method according to any one of [5] to [17] above, wherein the organic solvent is an ether solvent.
[20] One embodiment of the present invention relates to the method according to any one of [5] to [17] above, wherein metal lithium and an amine are allowed to act on the morphinan derivative represented by the General Formula (I) in absence of an organic solvent.
[21] One embodiment of the present invention relates to the method according to any one of [5] to [20] above, wherein a reaction temperature is -10°C to 120°C.
[22] One embodiment of the present invention relates to the method according to any one of [5] to [20] above, wherein a reaction temperature is -5°C to 105°C.
[23] One embodiment of the present invention relates to the method according to any one of [5] to [22] above, wherein a metal hydride is present in a reaction system.
[24] One embodiment of the present invention relates to the method according to [23] above, wherein the metal hydride is selected from lithium hydride, sodium hydride, potassium hydride, calcium hydride, lithium borohydride, sodium borohydride, diisobutylaluminum hydride, and lithium aluminum hydride.
[25] One embodiment of the present invention relates to a method for producing a morphinan derivative represented by General Formula (V) below: 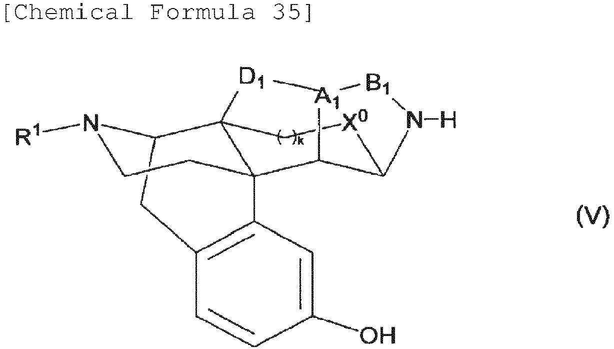
   wherein R¹ represents a hydrogen atom or any substituent,
   k represents 1 or 2,
   X⁰ represents a carbon atom optionally having a substituent, a nitrogen atom optionally having a substituent, or an oxygen atom,
   A₁ indicates CH or N,
   B₁ indicates an alkylene chain having 1 to 3 carbon atoms and optionally having a substituent, and the alkylene chain, together with NR⁰, optionally forms an amide bond, and
   D₁ indicates CH₂, NR⁰⁵, O, or S wherein R⁰⁵ represents a hydrogen atom or any substituent; or
   a salt, a tautomer, a stereoisomer, or an isotope of the morphinan derivative, comprising the step of:
      allowing metal lithium and an amine to act on a morphinan derivative represented by General Formula (IV) below: 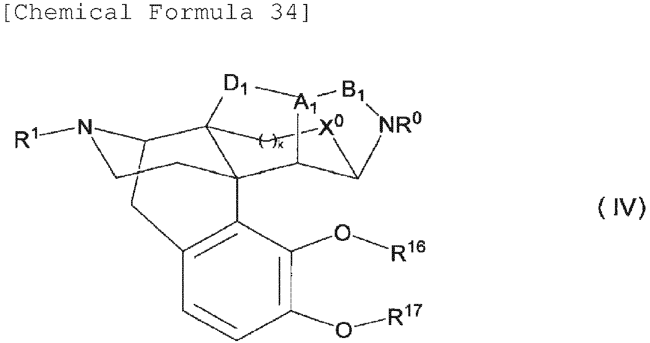
      wherein R¹ have a meaning same as above,
      R¹⁶ represents a C₆₋₁₀ aryl group optionally having a substituent or a heteroaryl group optionally having a substituent,
      R¹⁷ represents a C₁₋₁₀ alkyl group, an aralkyl group optionally having a substituent, an alkenyl group optionally having a substituent, or a silyl protecting group,
      k, X⁰, A₁, B₁, and D₁ have a meaning same as above, and
      R⁰ represents a hydrogen atom, an aralkyl group optionally having a substituent, or a heteroarylalkyl group optionally having a substituent; or
      a tautomer, a stereoisomer, or an isotope of the morphinan derivative in presence or absence of an organic solvent.
[26] One embodiment of the present invention relates to the method according to [25] above, wherein k is 1, and X⁰ is CH₂.
[27] One embodiment of the present invention relates to the method according to [25] or [26] above, wherein B₁ and D₁ are CH₂, and A₁ is CH.
[28] One embodiment of the present invention relates to the method according to any one of [25] to [27] above, wherein R¹ is a hydrogen atom, a methyl group, a cyclopropylmethyl group, a cyclobutylmethyl group, a benzyl group, or an allyl group.
[29] One embodiment of the present invention relates to the method according to any one of [25] to [28] above, wherein R¹ is a hydrogen atom.
[30] One embodiment of the present invention relates to the method according to any one of [25] to [29] above, wherein R¹⁶ is a C₆₋₁₀ aryl group optionally having a substituent.
[31] One embodiment of the present invention relates to the method according to any one of [25] to [29] above, wherein R¹⁶ is a phenyl group.
[32] One embodiment of the present invention relates to the method according to any one of [25] to [31] above, wherein R¹⁷ is a methyl group.
[33] One embodiment of the present invention relates to the method according to any one of [25] to [32] above, wherein R⁰ is a hydrogen atom or a benzyl group.
[34] One embodiment of the present invention relates to the method according to any one of [25] to [33] above, wherein the amine is liquid ammonia, a primary amine, or a secondary amine.
[35] One embodiment of the present invention relates to the method according to any one of [25] to [33] above, wherein the amine is a primary amine.
[36] One embodiment of the present invention relates to the method according to any one of [25] to [33] above, wherein the amine is a primary amine represented by General Formula (III) below: 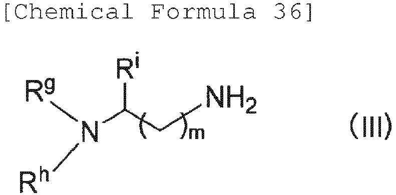 wherein R^{g}, R^{h}, and Rⁱ are same or different, and represent a hydrogen atom or a C₁₋₆ alkyl group optionally having a substituent or any two of R^{g}, R^{h}, and Rⁱ together optionally form a ring, and m represents an integer of 1 to 5.
[37] One embodiment of the present invention relates to the method according to any one of [25] to [36] above, wherein metal lithium is used in an amount of 2 to 20 equivalents relative to one functional group to be reduced in the morphinan derivative represented by the General Formula (IV) .
[38] One embodiment of the present invention relates to the method according to any one of [25] to [37] above, wherein the organic solvent is an aromatic hydrocarbon solvent, a lower alcohol solvent, or an ether solvent.
[39] One embodiment of the present invention relates to the method according to any one of [25] to [37] above, wherein the organic solvent is an ether solvent.
[40] One embodiment of the present invention relates to the method according to any one of [25] to [37] above, wherein metal lithium and an amine are allowed to act on the morphinan derivative represented by the General Formula (IV) in absence of an organic solvent.
[41] One embodiment of the present invention relates to the method according to any one of [25] to [40] above, wherein a reaction temperature is -10°C to 120°C.
[42] One embodiment of the present invention relates to the method according to any one of [25] to [40] above, wherein a reaction temperature is -5°C to 105°C.
[43] One embodiment of the present invention relates to the method according to any one of [25] to [42] above, wherein a metal hydride is present in a reaction system.
[44] One embodiment of the present invention relates to the method according to [43] above, wherein the metal hydride is selected from lithium hydride, sodium hydride, potassium hydride, calcium hydride, lithium borohydride, sodium borohydride, diisobutylaluminum hydride, and lithium aluminum hydride.
[45] One embodiment of the present invention relates to a morphinan derivative represented by General Formula (VI) below: 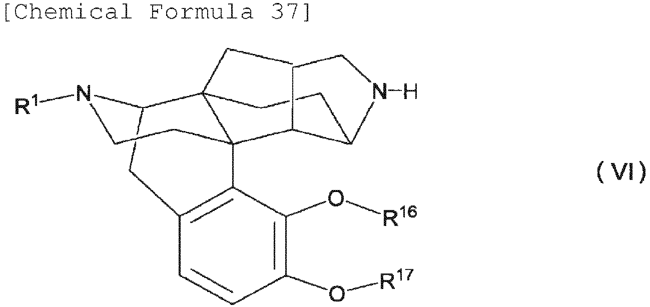
   wherein R¹ represents a hydrogen atom or any substituent,
   R¹⁶ represents a C₆₋₁₀ aryl group optionally having a substituent or a heteroaryl group optionally having a substituent, and
   R¹⁷ represents a C₁₋₁₀ alkyl group, an aralkyl group optionally having a substituent, an alkenyl group optionally having a substituent, or a silyl protecting group; or
   a tautomer, a stereoisomer, or an isotope of the morphinan derivative.
[46] One embodiment of the present invention relates to the morphinan derivative according to [45] above; or a tautomer, a stereoisomer, or an isotope of the morphinan derivative, wherein R¹ is a hydrogen atom, a methyl group, a cyclopropylmethyl group, a cyclobutylmethyl group, a benzyl group, or an allyl group.
[47] One embodiment of the present invention relates to the morphinan derivative according to [45] or [46] above; or a tautomer, a stereoisomer, or an isotope of the morphinan derivative, wherein R¹ is a hydrogen atom.
[48] One embodiment of the present invention relates to the morphinan derivative according to any one of [45] to [47] above; or a tautomer, a stereoisomer, or an isotope of the morphinan derivative, wherein R¹⁶ is a C₆₋₁₀ aryl group optionally having a substituent.
[49] One embodiment of the present invention relates to the morphinan derivative according to any one of [45] to [47] above; or a tautomer, a stereoisomer, or an isotope of the morphinan derivative, wherein R¹⁶ is a phenyl group.
[50] One embodiment of the present invention further relates to the morphinan derivative according to any one of [45] to [49] above; or a tautomer, a stereoisomer, or an isotope of the morphinan derivative, wherein R¹⁷ is a methyl group.

### Advantageous Effects of Invention

According to the present invention, it is possible to provide a method for converting a biphenyl ether form having an ortho-methoxy group into a monophenol derivative in one step. The method provided by the present invention can be applied to a compound having a morphinan skeleton.

### Description of Embodiments

One embodiment of the present invention relates to a production method of a phenol derivative, and is applicable to, for example, a compound represented by the General Formula (A), all biphenyl ether forms having an alkoxy group or the like at an ortho position, and all morphinan derivatives having a phenoxy group or the like optionally having a substitution at the 4-position, and the present invention will be described in more detail below. In the present specification, the term "isotope" means a compound labeled with a radioisotope element for each compound.

### <1.> Compound represented by General Formula (A) or General Formula (B)

R^{q1}, R^{q2}, R^{q3}, and R^{q4} in the General Formulae (A) and (B) are same or different, and examples thereof include a hydrogen atom; any substituent such as a C₁₋₁₀ alkyl group optionally having a substituent such as a halogen atom; a halogen atom; an amino group optionally having a substituent; C₆₋₁₀ aryl group optionally having a substituent; and an alkenyl group optionally having a substituent.

Examples of a case in which adjacent two of R^{q1}, R^{q2}, R^{q3}, and R^{q4} form a ring include a case in which R^{q1} and R^{q2} together form a 6-membered ring, and a benzene ring to which R^{q1} and R^{q2} are bonded forms a part of a morphinan ring.

Examples of the C₆₋₁₀ aryl group optionally having a substituent of R^{p1} in the General Formula (A) include C₆₋₁₀ aryl groups optionally having a substituent such as a halogen atom, an alkyl group having 1 to 6 carbon atoms, an amino group optionally having a substituent, and an alkoxy group having 1 to 6 carbon atoms, and preferably include a phenyl group.

Examples of the lower alkyl group of R^{p2} in the General Formula (A) include an alkyl group having 1 to 6 carbon atoms, the lower alkyl group is preferably a methyl group and the like, examples of the aralkyl group optionally having a substituent include a benzyl group, examples of the alkenyl group optionally having a substituent include an allyl group and the like, examples of the silyl protecting group include a t-butyldimethylsilyl group and the like, and examples of R^{p2} preferably include a methyl group.

### <2.> Compound represented by General Formula (I), General Formula (II), or General Formula (III)

R¹ to R¹⁵, R^{a}, and R^{b} in the General Formula (I) and the General Formula (II) are a hydrogen atom or any substituent, and preferable examples thereof include the following.

Examples of R¹ include a hydrogen atom, a C₁₋₁₀ alkyl group optionally having a substituent, a C₃₋₆ cycloalkyl group optionally having a substituent, a cycloalkylalkyl group optionally having a substituent (the cycloalkyl moiety has 3 to 6 carbon atoms and the alkylene moiety indicates 1 to 5 carbon atoms), an aralkyl group optionally having a substituent (the aryl moiety has 6 to 10 carbon atoms and the alkylene moiety indicates 1 to 5 carbon atoms), a heteroarylalkyl group optionally having a substituent (the heteroaryl contains 1 to 4 heteroatoms that are same or different and selected from N, 0, and S as ring-constituting atoms, and the alkylene moiety indicates 1 to 5 carbon atoms), a C₂₋₆ alkenyl group optionally having a substituent, a C₆₋₁₀ aryl group optionally having a substituent, a heteroaryl group optionally having a substituent, and an amino protecting group.

R² to R¹⁵ in the General Formulae (I) and (II) are same or different, and examples thereof include a hydrogen atom, a C₁₋₁₀ alkyl group optionally having a substituent, a C₁₋₁₀ alkoxy group optionally having a substituent, a halogen atom, an amino group optionally having a substituent, a C₂₋₆ alkenyl group optionally having a substituent, a C₆₋₁₀ aryl group optionally having a substituent, and a hydroxy group.

R^{a} and R^{b} in the General Formulae (I) and (II) are same or different, and examples thereof include a hydrogen atom, a C₁₋₁₀ alkyl group optionally having a substituent, a halogen atom, an amino group optionally having a substituent, a C₂₋₆ alkenyl group optionally having a substituent, and a C₆₋₁₀ aryl group optionally having a substituent.

R^{c0} and R^{d0} in C=CR^{c0}R^{d0} of X, and R^{c} and R^{d} in C=CR^{c}R^{d} formed by R¹⁰ and R¹¹, together with a carbon atom to which R¹⁰ and R¹¹ are bonded in the General Formulae (I) and (II) are same or different, and examples thereof include a hydrogen atom, a C₁₋₁₀ alkyl group optionally having a substituent, C₃₋₁₀ alkenyl group optionally having a substituent, an aryl group optionally having a substituent, and a heteroaryl group optionally having a substituent.

Examples of the C₁₋₁₀ alkyl group in the C₁₋₁₀ alkyl group optionally having a substituent of R¹ to R¹⁵, R^{a}, R^{b}, R¹⁷, R^{c}, R^{d,} Rⁱ, Cₓ, and C_{y} in the General Formulas (I), (II), and (III) include a methyl group, an ethyl group, a propyl group, an isopropyl group, a butyl group, an isobutyl group, a tert-butyl group, a pentyl group, and a hexyl group, preferably include linear or branched C₁₋₆ alkyl groups such as a methyl group, an ethyl group, a propyl group, an isopropyl group, a butyl group, an isobutyl group, and a tert-butyl group, and more preferably include a methyl group.

Examples of the C₃₋₆ cycloalkyl group in the C₃₋₆ cycloalkyl group optionally having a substituent of R¹ in the General Formulae (I) and (II) include a cyclopropyl group, a cyclobutyl group, a cyclopentyl group, and a cyclohexyl group, and preferably include a cyclopropyl group.

Examples of the cycloalkyl group in the cycloalkylalkyl group optionally having a substituent (the number of carbon atoms of the cycloalkyl moiety is 3 to 6, and the number of carbon atoms of the alkylene moiety indicates 1 to 5) of R¹ in the General Formulas (I) and (II) include a methyl group and an ethyl group substituted with a C₃₋₆ cycloalkyl group such as a cyclopropyl group, a cyclobutyl group, a cyclopentyl group, and a cyclohexyl group, preferably include a cyclopropylmethyl group, a cyclopropylethyl group, a cyclobutylmethyl group, and a cyclobutylethyl group, and the cycloalkyl group is more preferably a cyclopropylmethyl group.

Examples of the substituent in the C₁₋₁₀ alkyl group optionally having a substituent of R¹ to R¹⁵, R^{a}, R^{b}, R¹⁷, R^{c}, R^{d}, Rⁱ, Cₓ, and C_{y} in the General Formulas (I), (II), and (III), the C₃₋₆ cycloalkyl group optionally having a substituent of R¹, and the cycloalkylalkyl group optionally having a substituent include linear or branched C₁₋₆ alkyl groups such as a methyl group, an ethyl group, a propyl group, and an isopropyl group, halogenated methyl groups such as a fluoromethyl group, a difluoromethyl group, and a trifluoromethyl group, halogen atoms such as a fluorine atom and a chlorine atom, a hydroxy group, an amino group optionally having a substituent, and acyl groups such as an acetyl group, a cyclopropylcarbonyl group, and a benzoyl group.

Examples of the aralkyl group in the aralkyl group optionally having a substituent of the R¹ and R¹⁷ include a methyl group and an ethyl group that are substituted with phenyl or naphthyl and have the number of carbon atoms of the aryl moiety of 6 to 10 and the number of carbon atoms of the alkylene moiety of 1 to 5 and preferably include a methyl group substituted with phenyl (that is, a benzyl group) and an ethyl group substituted with phenyl (that is, a phenethyl group).

Examples of the heteroaryl group in the heteroarylalkyl group optionally having a substituent represented by the R¹ include a heteroaryl containing 1 to 4 heteroatoms selected from a nitrogen atom, an oxygen atom, and a sulfur atom as ring-constituting atoms, and examples of the heteroarylalkyl group include monocyclic heteroarylalkyl groups such as a (pyridine-2-yl)methyl group, a (pyridine-3-yl)methyl group, a (pyridine-4-yl)methyl group, a 2-(pyridine-2-yl)ethyl group, a (furan-2-yl)methyl group, a (furan-3-yl)methyl group, a (imidazole-2-yl)methyl group, a (imidazole-4-yl)methyl group, a (imidazole-5-yl)methyl group, a (thiazole-2-yl)methyl group, a (thiazole-4-yl)methyl group, a (thiazole-5-yl)methyl group, a (thiophene-2-yl)methyl group, and a 2-(thiophene-2-yl)ethyl group, and bicyclic heteroarylalkyl groups such as a (quinoline-3-yl)methyl group and a (indole-3-yl)methyl group.

Examples of the alkenyl group in the alkenyl group optionally having a substituent of the R¹ and R¹⁷ include a C₃₋₆ linear or branched alkenyl group, and include alkenyl groups such as an allyl group, a 2-butenyl group, a 3-butenyl group, a 2-pentenyl group, a 3-pentenyl group, a 4-pentenyl group, a 2-hexenyl group, a 3-hexenyl group, a 4-hexenyl group, and a 5-hexenyl group.

Examples of the C₆₋₁₀ aryl group in the C₆₋₁₀ aryl group optionally having a substituent of the R¹ and R¹⁶ include a phenyl group and a naphthyl group, and preferably include a phenyl group.

Examples of the heteroaryl group in the heteroaryl group optionally having a substituent in the R¹ and R¹⁶ include a furyl group, a thienyl group, an imidazolyl group, a thiazolyl group, a thiadiazolyl group, an oxazolyl group, an oxadiazolyl group, a pyridyl group, a pyrimidyl group, a pyridazyl group, a pyrazinyl group, and a tetrazolyl group, and the heteroaryl group is preferably a thienyl group, a pyridyl group, or a tetrazolyl group.

Examples of the C₁₋₆ alkoxy group in the C₁₋₆ alkoxy group optionally having a substituent of the R² to R¹⁵ include a methoxy group, an ethoxy group, a propoxy group, an iso-propoxy group, a butoxy group, and an iso-butoxy group, and preferably include a methoxy group.

Examples of the halogen atom of the R² to R¹⁵, R^{a}, and R^{b} include a fluorine atom, a chlorine atom, a bromine atom, and an iodine atom, preferably include a fluorine atom and a chlorine atom, and more preferably include a fluorine atom.

Examples of the silyl protecting group of the R¹⁷ include a trimethylsilyl group, a triethylsilyl group, a tert-butyldimethylsilyl group, a triisopropylsilyl group, and a tert-butyldiphenylsilyl group, and preferably include a tert-butyldimethylsilyl group and a triisopropylsilyl group.

Examples of the amino protecting group of R¹ include carbamate protecting groups such as a methoxycarbonyl group, an ethoxycarbonyl group, a tert-butoxycarbonyl group, a tert-amyloxycarbonyl group, a 2,2,2-trichloroethoxycarbonyl group, a benzyloxycarbonyl group, a p-chlorobenzyloxycarbonyl group, a p-methoxybenzylcarbonyl group, a p-nitrobenzyloxycarbonyl group, a p-phenylazobenzyloxycarbonyl group, a p-methoxyphenylazobenzyloxycarbonyl group, a 3,5-dimethoxybenzyloxycarbonyl group, a 3,4,5-trimethoxybenzyloxycarbonyl group, a p-biphenylisopropyloxycarbonyl group, a diisopropylmethyloxycarbonyl group, a 2-(trimethylsilyl)ethoxycarbonyl group, and a 9-fluorenylmethyloxycarbonyl group; sulfonamide protecting groups such as a p-toluenesulfonyl group and a 2-nitrobenzenesulfonyl group; imide protecting groups such as a phthaloyl group; acyl protecting groups such as an acetyl group and a trifluoroacetyl group; and C₇₋₁₉ aralkyls such as a benzyl group, a phenylethyl group, a phenylpropyl group, a trityl group, and a naphthylmethyl group.

Examples of the saturated hydrocarbon ring optionally having a substituent formed by R⁰³ and R¹⁰ bonding to each other in the General Formulae (I) and (II) include those having 3 to 8 carbon atoms such as cyclopropane, cyclobutane, cyclopentane, cyclohexane, and cycloheptane, and preferably include cyclopentane and cyclohexane.

Examples of the saturated heterocycle optionally having a substituent formed by R⁰³ and R¹⁰ bonding to each other in the General Formulas (I) and (II) include cyclic amines of a 4 to 6 membered ring such as azetidine, pyrrolidine, piperidine, piperazine, and morpholine, cyclic ethers such as tetrahydrofuran, tetrahydrothiophene, and dioxane, and cyclic thioethers, the saturated heterocycle is preferably a cyclic amine, and examples thereof still more preferably include pyrrolidine and piperidine.

Examples of the unsaturated hydrocarbon ring optionally having a substituent formed by R⁰¹ and R¹⁰ bonding to each other in the General Formulae (I) and (II) include cycloalkenes having 5 to 8 carbon atoms such as cyclopentene and cyclohexene, and aromatic hydrocarbons having 6 to 10 carbon atoms such as benzene and naphthalene, and preferably include benzene.

Examples of the unsaturated heterocycle optionally having a substituent formed by R⁰¹ and R¹⁰ bonding to each other in the General Formulas (I) and (II) include 5-membered rings such as pyrrole, furan, imidazole, oxazole, isoxazole, oxadiazole, thiophene, thiazole, isothiazole, and thiadiazole, and 6-membered rings such as pyridine, pyrimidine, pyridazine, and pyrazine, and preferably include furan, thiophene, thiazole, and pyridine.

Examples of the saturated heterocycle optionally having a substituent formed by R⁰² and R¹⁰ bonding to each other in the General Formulas (I) and (II) include cyclic amines of a 4 to 6 membered ring such as azetidine, pyrrolidine, piperidine, piperazine, and morpholine, and preferably include pyrrolidine and piperidine.

Examples of the unsaturated hydrocarbon ring optionally having a substituent formed by R⁰³ and R¹⁰ bonding to each other in the General Formulae (I) and (II) include cycloalkenes having 5 to 8 carbon atoms such as cyclopentene and cyclohexene.

Examples of the unsaturated heterocycle optionally having a substituent formed by R⁰³ and R¹⁰ bonding to each other in the General Formulas (I) and (II) include tetrahydropyridine and dihydropyran.

The lactam ring optionally having a substituent formed by R⁰¹, R⁰² or R⁰³ and R¹⁰ bonding to each other in the General Formulas (I) and (II), the ring formed together with an alkylene chain that is formed by any two groups of R⁸, R¹⁰, and R¹⁴ bonding to each other, and the ring formed together with the remaining one group can include a δ-lactam and a γ-lactam.

Examples of those capable of being fused with a saturated hydrocarbon ring, a saturated heterocycle, an unsaturated hydrocarbon ring, an unsaturated heterocycle, and a lactam ring optionally having a substituent, which are formed by R⁰¹, R⁰² or R⁰³ and R¹⁰ bonding to each other in the General Formulas (I) and (II) include a saturated hydrocarbon ring, a saturated heterocycle, an unsaturated hydrocarbon ring, and an unsaturated heterocycle, and examples of those formed by fusing preferably include quinoline, indole, benzofuran, and benzothiophene.

Among saturated hydrocarbon rings optionally having a substituent, a saturated heterocycle optionally having a substituent, an unsaturated hydrocarbon ring optionally having a substituent, an unsaturated heterocycle optionally having a substituent, and a lactam ring optionally having a substituent, which are formed by the R⁰¹, R⁰² or R⁰³ and R¹⁰ bonding to each other, and the fused rings, an unsaturated heterocycle optionally having a substituent and a fused unsaturated heterocycle are preferable, and as a compound represented by the General Formula (I), for example, compounds represented by the following Chemical Formulae are preferable. 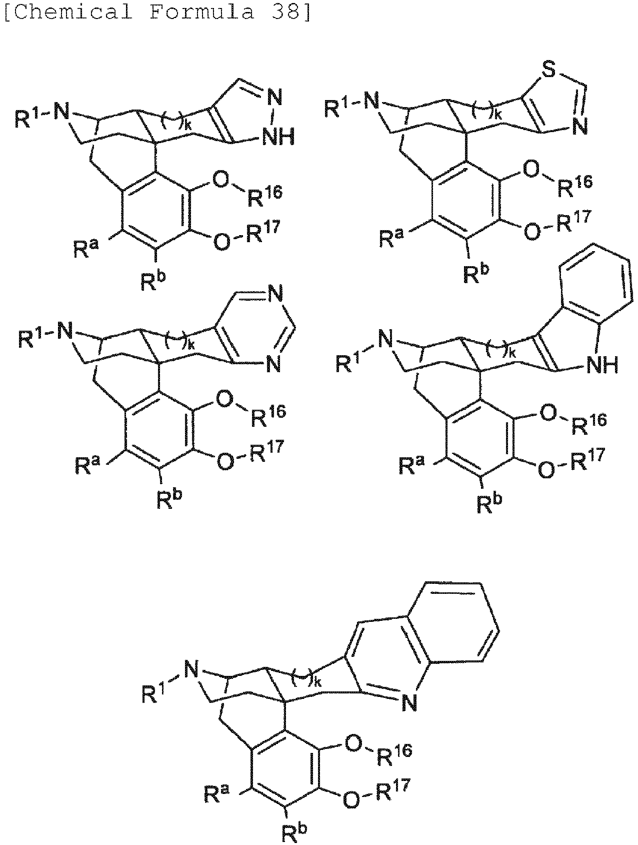 wherein R¹, R¹⁶, R¹⁷, R^{a}, R^{b}, and k have a meaning same as above.

For R⁸, R¹⁰, and R¹⁴, any two groups selected from these can be bonded to each other to form a C₁₋₄ alkylene chain, the alkylene chain can be substituted with a substituent, a carbon atom that constitutes the alkylene chain can be replaced with a heteroatom such as a sulfur atom, an oxygen atom, and a nitrogen atom, the alkylene chain can further have a double bond or an amide bond in the middle, and the rest other than the two selected groups can be bonded to the alkylene chain.

For R⁸, R¹⁰, and R¹⁴, examples of the morphinan derivative to which R¹⁰ and R¹⁴ are bonded include, but are not limited to, that shown in the following (a), and examples of the morphinan derivative in which R⁸ is further bonded to the group formed by R¹⁰ and R¹⁴ bonding to each other include, but are not limited to, that shown in the following (b).

(a) Compound in which R¹⁰ and R¹⁴ are bonded to each other to form a 5 to 8 membered ring. 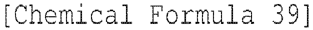 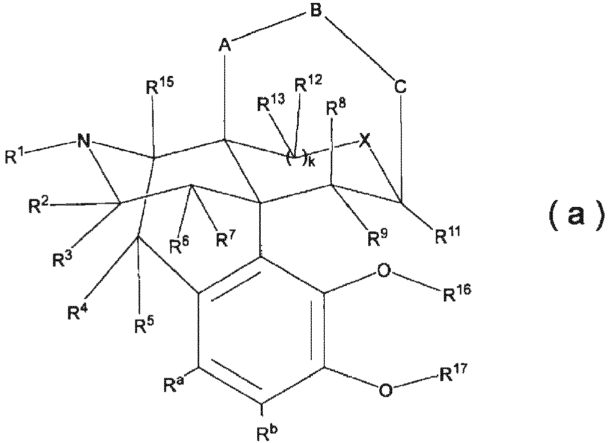 wherein R¹ to R⁹, R¹¹ to R¹³, R¹⁵ to R¹⁷, R^{a}, R^{b}, k, and X have a meaning same as above. A and C represent CH₂, NR^{t} wherein R^{t} indicates an amino protecting group, an aralkyl group optionally having a substituent, or a heteroaryl group optionally having a substituent, or an oxygen atom, and B represents a bond or an alkylene chain having 1 to 3 carbon atoms optionally having a substituent).

Specific examples thereof include the following compounds. The description of substituents other than the indicated substituents is omitted.

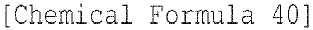

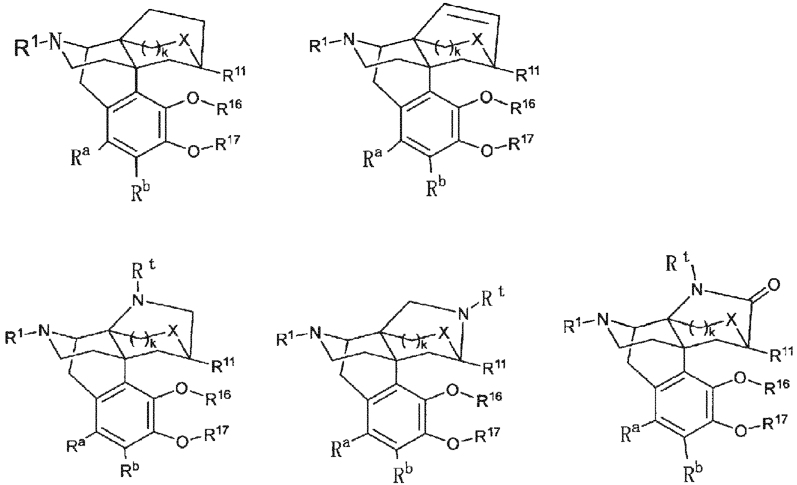

### (b) Compound in which R⁸, R¹⁰ and R¹⁴ are bonded to each other

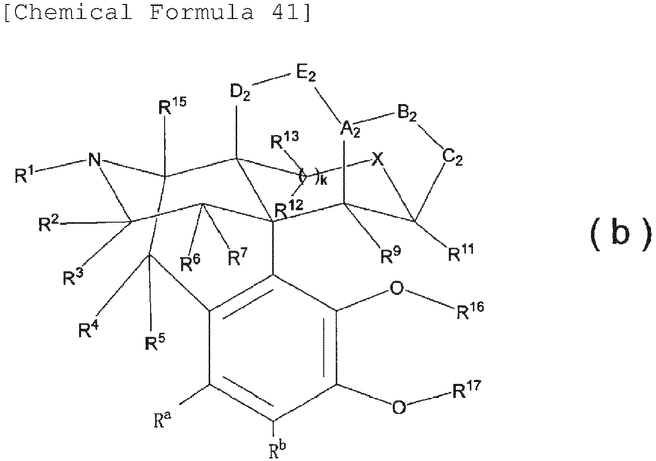

wherein R¹ to R⁷, R⁹, R¹¹ to R¹³, R¹⁵ to R¹⁷, R^{a}, R^{b}, k, and X have a meaning same as above. A₂ indicates CH or N, C₂ and D₂ indicate CH₂, NR^{t}, O, or S, B₂ and E₂ indicate a bond or an alkylene chain having 1 to 3 carbon atoms optionally having a substituent, and examples of the substituent include a C₁₋₆ alkyl group, a C₁₋₆ alkoxy group, a cycloalkyl group optionally having a substituent, an aryl group optionally having a substituent, a heteroaryl group optionally having a substituent, a halogen atom, a hydroxy group, an amino group optionally having a substituent such as a C₁₋₆ alkylamino group, =O, and =S.

Specific examples thereof include the following compounds. The description of substituents other than the indicated substituents is omitted.

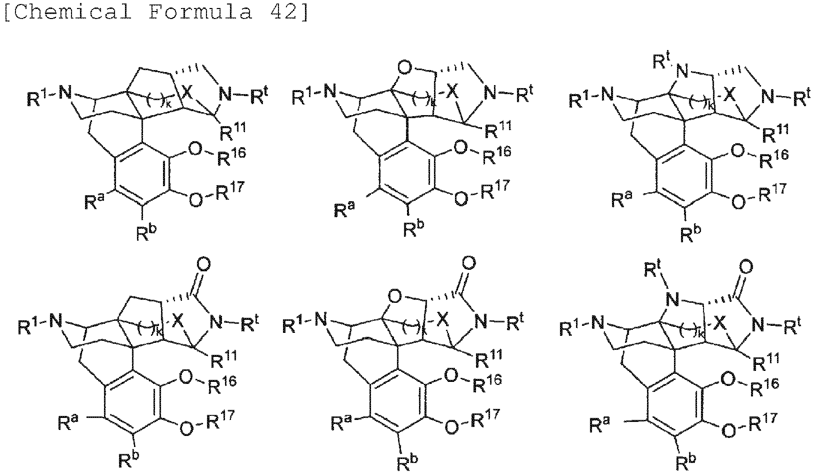

From another viewpoint of the present invention, when a morphinan derivative has a nitrogen atom as a ring-constituting atom and the nitrogen atom is protected by an aralkyl group or a heteroarylalkyl group (particularly desirably a benzyl group) as in the derivative described in Patent Literature 11, all deprotection reactions can be performed in one step, including the dephenoxylation reaction at the 4-position and the removal of the alkyl protecting group of phenol at the 3-position (particularly preferably, removal of a benzyl group or a methyl group) by applying the present invention. More specific examples of the morphinan derivative to which the present invention can be applied in the General Formula (I) include a derivative indicated by the following General Formula (c). 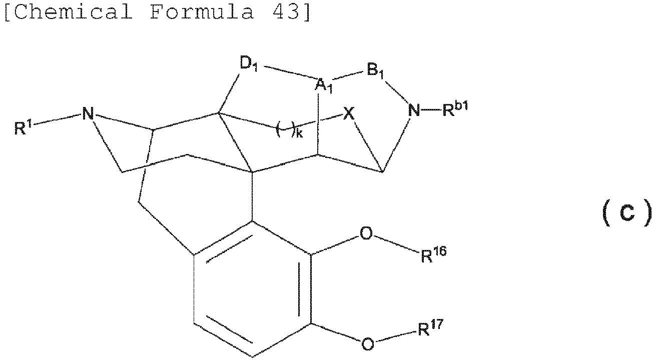 wherein R¹, R¹⁶, R¹⁷, A₁, B₁, D₁, k, and X have a meaning same as in the General Formula (IV). Examples of R^{b1} include an aralkyl group optionally having a substituent and a heteroarylalkyl group optionally having a substituent, and preferably include an aralkyl group optionally having a substituent.

Specific examples thereof include the following compounds. The description of substituents other than the indicated substituents is omitted.

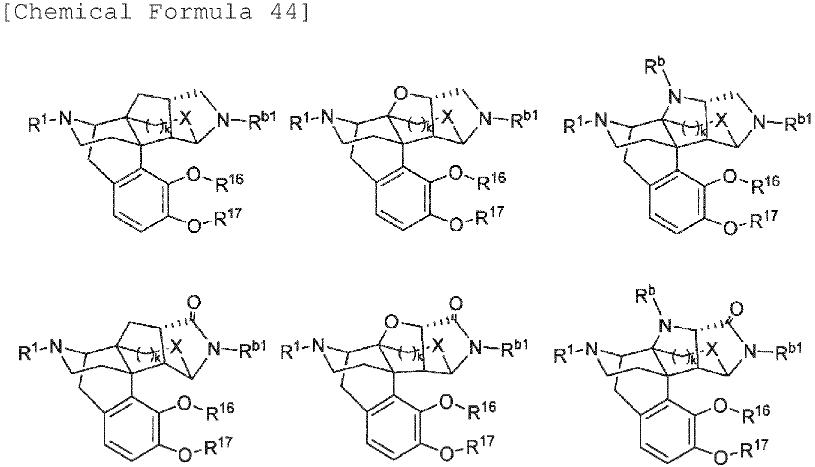

Among the derivative represented by the General Formula (a), (b) or (c), a derivative of the formula (b) or (c) is preferable, and a derivative of the formula (c) is more preferable.

The compound represented by the General Formula (I) can be obtained with reference to the method described in

Patent Literature 3, the methods described in Patent Literatures 1, 2, and 4 to 11, and Non-Patent Literatures 1 to 16, and the like.

Examples of the cyclic ketal in the cyclic ketal group optionally having a substituent, which is formed together with the carbon to which R¹⁰ and R¹¹ are bonded include dioxolane and dioxane.

k indicates an integer of 1 or 2, and is preferably 1.

m indicates an integer of 1 to 5, and is preferably 1 or 2.

A double line consisting of a solid line and a broken line indicates a single bond or a double bond, and a single bond is preferable.

Examples of the amino group optionally having a substituent include an amino group, a linear or branched C₁₋₁₀ alkylamino group, an N- (linear or branched C₁₋₁₀ alkyl group)-N-(linear or branched C₁₋₁₀ alkyl group) amino group, an acylamino group, and a protected amino group.

Examples of a substituent in the case where the substituent is not specified herein include linear or branched C₁₋₆ alkyl groups such as a methyl group, an ethyl group, a propyl group, and an isopropyl group; halogenated C₁₋₁₀ alkyl groups such as a fluoromethyl group, a difluoromethyl group, and a trifluoromethyl group; linear or branched C₁₋₆ alkoxy groups such as a methoxy group, an ethoxy group, a propoxy group, and an iso-propoxy group; halogenated C₁₋₁₀ alkoxy groups such as a fluoromethoxy group, a difluoromethoxy group, a trifluoromethoxy group, and a 2,2,2-trifluoroethoxy group; halogen atoms such as a fluorine atom and a chlorine atom; amino groups optionally having a substituent; acyl groups such as an acetyl group, a cyclopropylcarbonyl group, and a benzoyl group; C₂₋₆ alkenyl groups such as a 2-propenyl group; and a cyano group, and a hydroxy group.

### <3.> Compound represented by General Formula (IV) or General Formula (V)

R¹ and k in the General Formulae (IV) and (V) have a meaning same as those described in <2.> above, and specific examples thereof are also same.

R¹⁶ and R¹⁷ in the General Formula (IV) have a meaning same as those described in <2.> above, and specific examples thereof are also same.

Examples of X⁰ in the General Formulas (IV) and (V) include CR²¹R²², NR²³, and an oxygen atom (R²¹ to R²³ represent a hydrogen atom, an alkyl group having 1 to 6 carbon atoms, or the like), and preferably include CH₂.

As A¹ in the General Formulae (IV) and (V), CH is preferable.

B¹ in the General Formulae (IV) and (V) indicates an alkylene chain having 1 to 3 carbon atoms and optionally having a substituent such as a halogen atom, the alkylene chain can form an amide bond together with NR⁰, and examples thereof preferably include CH₂.

Examples of D¹ in the General Formulae (IV) and (V) include CH₂, NR⁰⁵, O, and S wherein R⁰⁵ represents a hydrogen atom, an alkyl group having 1 to 6 carbon atoms, or the like, and preferably include CH₂.

Examples of R⁰ in the General Formula (IV) include a hydrogen atom; aralkyl groups optionally having a substituent such as a halogen atom, an alkyl group having 1 to 6 carbon atoms, and an alkoxy group having 1 to 6 carbon atoms (for example, a C₁₋₃ alkyl group substituted with a C₆₋₁₀ aryl group); and a heteroarylalkyl group optionally having a substituent such as a halogen atom, an alkyl group having 1 to 6 carbon atoms, and an alkoxy group having 1 to 6 carbon atoms (for example, a C₁₋₃ alkyl group substituted with a 5 to 7 membered heterocycle such as thiazole), and preferably include a hydrogen atom and a benzyl group.

The compound represented by the General Formula (IV) can be obtained with reference to the method described in

Patent Literature 3, the methods described in Patent Literatures 1, 2, and 4 to 11, and Non-Patent Literatures 1 to 16, and the like.

### <4.> Compound represented by General Formula (VI)

R¹, R¹⁶, and R¹⁷ in General Formula (VI) have a meaning same as those described in <2.> above, and specific examples thereof are also same.

The compound represented by General Formula (VI) can be obtained, for example, by (i) debenzylation of the compound represented by the General Formula (IV) wherein R⁰ represents a benzyl group by catalytic reduction in the presence of Pd/C, or (ii) allowing phenyl chloroformate to act on the compound represented by General Formula (IV) to produce a phenyl carbamate form, and then allowing a base to act thereon.

### <5.> Condition of reduction reaction

The morphinan derivative represented by the General Formula (II) can be produced by reacting metal lithium with the morphinan derivative represented by the General Formula (I) under the conditions of the Birch reduction or the Benkeser reduction.

As the metal lithium used in the method of the present invention, a commercially available metal lithium can be used, and for example, a wire-like metal lithium, a granular metal lithium, and a chip-like metal lithium are sold. A granular metal lithium or a chip-like metal lithium is preferable from the viewpoint of safety and ease of use.

In the method of the present invention, examples of the organic solvent include aromatic hydrocarbons such as benzene, toluene, and xylene; ethers such as diethyl ether, tert-butyl methyl ether, diisopropyl ether, tetrahydrofuran, dioxane, and ethylene glycol dimethyl ether; alcohols such as methanol, ethanol, isopropanol, and tert-butyl alcohol, and solvents such as acetone, acetonitrile, N,N-dimethylformamide, dimethylsulfoxide, and ethyl acetate, preferably include aromatic hydrocarbons and ethers, and still more preferably include tetrahydrofuran, benzene, toluene, and xylene. The reaction can also be performed under solvent-free conditions, that is, in the absence of an organic solvent. When an organic solvent is used, though the amount of the organic solvent used is not particularly limited, it is preferably in a range of 1 to 50 times by weight relative to the amount of the morphinan derivative represented by the General Formula (I). Because metal lithium is used, these organic solvents are preferably used after being purified by a known method such as distillation.

The reaction is usually performed at a temperature between -30°C and the boiling point of the organic solvent used, for example, between -30°C and 150°C, and can be preferably performed in the range of -10°C to 120°C, more preferably performed in the range of -5°C to 105°C, and still more preferably performed in the range of 70 to 95°C. The reaction terminates in 30 minutes to 24 hours, and preferably terminates in 1 hour to 20 hours.

In the method of the present invention, the three functional group conversions of the dephenoxylation reaction at the 4-position, the dealkylation reaction of the alkoxy group at the 3-position, and the N-debenzylation reaction of the N-benzyl group under the Birch reduction or the Benkeser reduction conditions with metal lithium can be performed by One-Pot, and the amount of metal lithium used can be 2.0 to 50 equivalents, and can be preferably 2.0 to 20 equivalents relative to the morphinan derivative represented by the General Formula (I) per functional group conversion.

Examples of the amine used in the method of the present invention include ammonia (for example, liquid ammonia), a primary amine, and a secondary amine. Examples of the primary amine include alkylamines such as methylamine, ethylamine, propylamine, isopropylamine, butylamine, isobutylamine, sec-butylamine, and tert-butylamine; primary amines represented by the General Formula (III) such as ethylenediamine and propylenediamine; and polyamines such as spermin, examples of the secondary amine include dialkylamines such as dimethylamine, diethylamine, and methylethylamine; and cyclic amines such as pyrrolidine, piperidine, piperazine, and morpholine, and the amine is preferably a primary amine, more preferably a primary amine represented by the General Formula (III), still more preferably ethylenediamine or propylenediamine, and most preferably ethylenediamine.

The amount of the amine used can be, for example, 1 to 20 mol, preferably 1 to 15 mol, and more preferably 1 to 10 mol relative to 1 mol of lithium in the reaction in the presence of an organic solvent.

In the reaction in the absence of an organic solvent, the amount of the amine used can be 5 to 50 mol, preferably 5 to 30 mol, more preferably 5 to 20 mol, and still more preferably 5 to 15 mol relative to 1 mol of lithium.

Examples of the metal hydride used in the method of the present invention include lithium hydride, sodium hydride, potassium hydride, calcium hydride, lithium borohydride, sodium borohydride, diisobutylaluminum hydride, and lithium aluminum hydride.

The amount of the metal hydride used can be 0.1 wt% to 20 wt%, and preferably 1 wt% to 10 wt% relative to the amount of the amine used.

Though the method of the present invention can be performed in an open system, it is preferably performed in an atmosphere of an inert gas such as nitrogen and argon.

Examples of performing the three functional group conversions by One-Pot include production of the compound represented by the General Formula (V) from the compound represented by the General Formula (IV) (for example, a compound in which R⁰ is a benzyl group or the like), and the method of the present invention described above can be similarly applied. The derivative represented by the General Formula (V) can also be effectively produced by applying the method of the present invention to the derivative (IV) represented by the General Formula (a compound in which R⁰ is a hydrogen atom).

Further, the reduction reaction from the compound represented by the General Formula (A) to the compound represented by the General Formula (B) can also be performed using the same method as the method for obtaining the morphinan derivative represented by General Formula (II) from the morphinan derivative represented by the General Formula (I) described above.

A phenol derivative represented by General Formula (B), or a salt, a tautomer, a stereoisomer, or an isotope of the phenol derivative; a morphinan derivative represented by General Formula (II), or a salt, a tautomer, a stereoisomer, or an isotope of the morphinan derivative; or the morphinan derivative represented by General Formula (V), or a salt, a tautomer, a stereoisomer, or an isotope of the morphinan derivative produced by the above-mentioned method can be isolated and/or purified by applying a method known per se, for example, solvent extraction, concentration, crystallization, precipitation, filtration, chromatography, or the like. When the production method of the present invention is applied as an industrial production method, chromatography is preferably not used as a purification method from the viewpoint of production efficiency.

Thus, one embodiment of the present invention is the production method, including at least one step selected from solvent extraction, concentration, crystallization, precipitation, and filtration, and not including a step of chromatography as a step of isolating and/or purifying a phenol derivative represented by General Formula (B), or a salt, a tautomer, a stereoisomer, or an isotope of the phenol derivative; a morphinan derivative represented by General Formula (II), or a salt, a tautomer, a stereoisomer, or an isotope of the morphinan derivative; or the morphinan derivative represented by General Formula (V), or a salt, a tautomer, a stereoisomer, or an isotope of the morphinan derivative.

By applying the isolation and/or purification step mentioned above, a phenol derivative represented by General Formula (B), or a salt, a tautomer, a stereoisomer, or an isotope of the phenol derivative; a morphinan derivative represented by General Formula (II), or a salt, a tautomer, a stereoisomer, or an isotope of the morphinan derivative; or the morphinan derivative represented by General Formula (V), or a salt, a tautomer, a stereoisomer, or an isotope of the morphinan derivative can be obtained, for example, with a purity of 75% or more, preferably with a purity of 80% or more, more preferably with a purity of 90% or more, and still more preferably with a purity of 95% or more, and with a yield of 30% or more, preferably with a yield of 40% or more, more preferably with a yield of 50% or more, and still more preferably with a yield of 60% or more.

In the present specification, a salt of the phenol derivative represented by General Formula (B), a salt of the morphinan derivative represented by General Formula (II), or a salt of the morphinan derivative represented by General Formula (V) means a conventional salt used in the field of organic chemistry, and examples thereof include salts of a base addition salt at the phenolic hydroxyl group when the derivative has a phenolic hydroxyl group, and an acid addition salt at the amino group or the basic heterocyclic group when the derivative has an amino group or a basic heterocyclic group.

Examples of the base addition salt include salts of alkali metals such as sodium and potassium; salts of alkaline earth metals such as calcium and magnesium; ammonium salts; and salts of organic amines such as trimethylamine, triethylamine, dicyclohexylamine, ethanolamine, diethanolamine, triethanolamine, procaine, and N,N'-dibenzylethylenediamine.

Examples of the acid addition salt include salts of inorganic acids such as hydrochloric acid, sulfuric acid, nitric acid, phosphoric acid, and perchloric acid; salts of organic acids such as acetic acid, formic acid, maleic acid, fumaric acid, tartaric acid, citric acid, ascorbic acid, and trifluoroacetic acid; and sulfonates such as methanesulfonic acid, isethionic acid, benzenesulfonic acid, and p-toluenesulfonic acid.

The salts can be mutually converted between the free form and the salt or between different salts by a method known per se before and after the isolation and/or purification step.

### Examples

Hereinafter, the present invention will be described in more detail with reference to Examples, Comparative Examples, and Test Examples, but the present invention is not limited thereto. For the naming of the compounds of Examples and the compounds of Reference Examples, the structural formulas drawn using ChemDraw ver. 15 manufactured by CambridgeSoft were converted into English names by a naming algorithm equipped in the same software, and then translated into Japanese.

### (Reference Example 1)

Production of (1S,3aR,5aS,6R,11bS,11cS)-3-benzyl-14-(cyclopropylmethyl)-10-methoxy-11-phenoxy-2,3,3a,4,5,6,7,11c-octahydro-1H-6,11b-(epiminoethano)-1,5a-methanonaphtho[1,2-e]indole free form and dihydrochloride

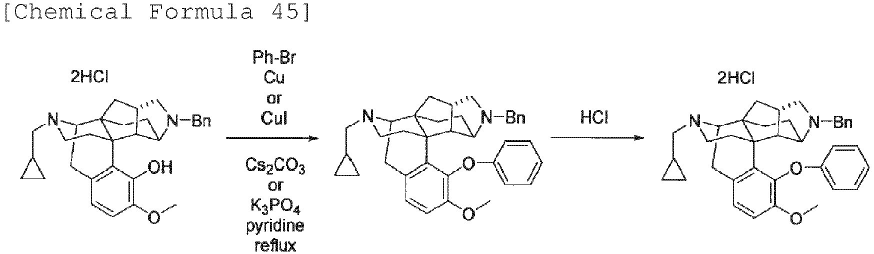

### (Production method in which copper powder is used)

In a 1000 mL round bottom flask, (1S,3aR,5aS,6R,11bS,11cS)-3-benzyl-14-(cyclopropylmethyl)-10-methoxy-2,3,3a,4,5,6,7,11c-octahydro-1H-6,11b-(epiminoethano)-1,5a-methanonaphtho[1,2-e]indole-11-ol dihydrochloride (27 g, 1 equivalent) was dissolved in pyridine (270 mL) under a nitrogen atmosphere. Cesium carbonate (97 g, 6 equivalents), bromobenzene (39 g, 5 equivalents), and copper powder (3.2 g, 1 equivalent) were added to the obtained solution, and the resulting mixture was heated and refluxed while being vigorously stirred. After 6 hours, the reaction was checked by HPLC, and the conversion rate was found to be 85%. Pyridine (270 mL), cesium carbonate (97 g, 6 equivalent), bromobenzene (39 g, 5 equivalents), and copper powder (3.2 g, 1 equivalent) were added to the reaction solution, and the resulting mixture was heated and refluxed one day while being vigorously stirred. The conversion rate was checked by HPLC, and was found to be 98%.

After cooling the reaction mixture to room temperature, the reaction mixture was filtered through a celite pad to remove insolubles. The pad was washed with ethyl acetate (500 mL) and methanol (500 mL), and the combined filtrate and washings were concentrated under reduced pressure. The obtained residue was dissolved in ethyl acetate (400 mL), washed twice with a saturated aqueous ammonium chloride solution (150 mL), and then washed twice with 6% aqueous ammonia (100 mL). The separated aqueous layer was washed twice with ethyl acetate (80 mL). The combined organic layers were dried over anhydrous sodium sulfate, filtered off, and then concentrated under reduced pressure to obtain a title compound crude free form (28 g, 103%).

The obtained crude free form was dissolved in a 1 : 1 mixture (3.5 L) of n-heptane and ethyl acetate, filtered through a short column packed with silica gel (140 g) using the same mixed solvent as an elution solvent, and then concentrated under reduced pressure to obtain a title compound free form (21.6 g).

The NMR spectrum was consistent with that reported in a compound 74 of Example 64 in Patent Literature 3.

The obtained free form was dissolved in ethyl acetate (150 mL), and then a 4 N hydrogen chloride/ethyl acetate solution (20 mL) was slowly added thereto at 0 to 5°C with stirring to obtain a suspension.

The resulting solid was collected by filtration to obtain a title compound dihydrochloride (21.6 g, 79%) as a light brown amorphous solid. Purity: 83%

¹H-NMR (400 MHz, CD₃OD) δ7.50 - 7.59 (m, 5H), 6.77 - 7.36 (m, 7H), 4.27 - 4.43 (m, 1H), 4.24 - 4.26 (m, 1H), 4.07 - 4.14 (m, 1H), 2.79 - 2.88 (m, 14H), 1.85 - 2.11 (m, 4H), 1.69 - 1.72 (m, 1H), 1.15 - 1.60 (m, 1H), 0.70 - 0.80 (m, 2H), 0.48 - 0.67 (m, 2H).

### (Production method in which catalytic amount of copper iodide is used)

Pyridine (1600 mL) was added to a reaction vessel filled with nitrogen at room temperature, and nitrogen gas was passed through pyridine for 10 minutes to replace oxygen in the solvent with nitrogen. Then, (1S,3aR,5aS,6R,11bS,11cS)-3-benzyl-14-(cyclopropylmethyl)-10-methoxy-2,3,3a,4,5,6,7,11c-octahydro-1H-6,11b-(epiminoethano)-1,5a-methanonaphtho[1,2-e]indole-11-ol dihydrochloride (173.9 g, 0.32 mol, 1 equivalent) and potassium phosphate (305.7 g, 1.44 mol, 4.5 equivalents) were added thereto at room temperature under a nitrogen atmosphere, and the mixture was heated to 95 to 105°C and stirred for 60 minutes. Cuprous iodide (12.2 g, 64 mmol, 0.2 equivalents) and bromobenzene (226.1 g, 151.7 mL, 1.44 mol, 4.5 equivalents) were added to the reaction solution with stirring at the same temperature, and the mixture was stirred at 110 to 120°C under a nitrogen atmosphere. After 6 hours, the conversion rate was found to be 85% by HPLC analysis, thus bromobenzene (8.4 mL, 0.25 equivalents) was added thereto, and the mixture was stirred for 14 hours. The conversion rate was found to be 99.4% by HPLC analysis.

The reaction solution was cooled to room temperature, the unnecessary substance was filtered off with a Seitz filter, and washed with ethyl acetate (450 mL). The collected filtrate and washings were concentrated under reduced pressure. Ethyl acetate (1800 mL) was added to and dissolved in the residue, and then 8% aqueous ammonia (1000 mL) was slowly added to adjust the pH to 10. The solution of the obtained two layers was stirred for 20 minutes and a dark purple aqueous layer was separated. The aqueous layer was extracted with ethyl acetate (800 mL) and the combined organic layers were washed with water (1200 mL, 1000 mL). The aqueous layer was filtered through a filter packed with silica gel 60 (125 g), and the filtrate was concentrated under reduced pressure. The residue was dissolved in ethyl acetate (1000 mL) and cooled to 5°C. A 15% hydrogen chloride/ethyl acetate solution (220 mL, 4 equivalents) was slowly added to the obtained solution. Heptane (2000 mL) was added to the obtained suspension, and the mixture was stirred at 0 to 10°C for 30 minutes. The obtained solid was collected by filtration, washed with a mixed solution of ethyl acetate and heptane (1 : 2, 200 mL x 2), decompressed, and dried at 40°C for 12 hours to obtain a dihydrochloride of the title compound (177.9 g, 89.7%) as a light brown amorphous solid. Purity: 92.4%

The NMR spectrum was consistent with that of the production method in which copper powder is used.

### (Reference Example 2)

Production of (1S,3aR,5aS,6R,11bS,11cS)-3-benzyl-11-(4-(tert-butyl)phenoxy)-14-(cyclopropylmethyl)-10-methoxy-2,3,3a,4,5,6,7,11c-octahydro-1H-6,11b-(epiminoethano)-1,5a-methanonaphtho[1,2-e]indole free form and dihydrochloride

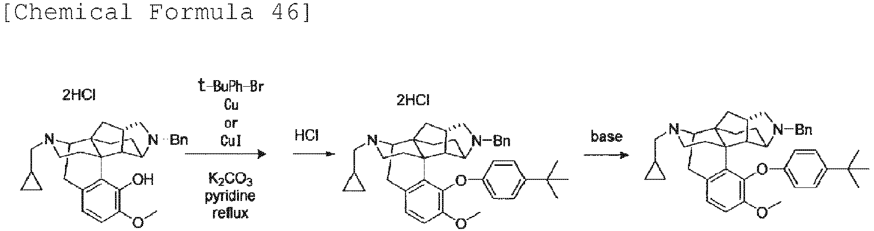

### (Production method in which copper powder is used)

Pyridine (45 L) was added to a 100 L reaction vessel at room temperature, (1S,3aR,5aS,6R,11bS,11cS)-3-benzyl-14-(cyclopropylmethyl)-10-methoxy-2,3,3a,4,5,6,7,11c-octahydro-1H-6,11b-(epiminoethano)-1,5a-methanonaphtho[1,2-e]indole-11-ol dihydrochloride (2.2 kg, 1 equivalent) and potassium carbonate (2.8 kg, 5 equivalents) were added thereto, and the mixture was stirred at a range of 95°C to 105°C for 60 minutes under a nitrogen atmosphere. Copper powder (259 g, 1 equivalent) and 1-bromo-4-(tert-butyl)benzene (4.31 kg, 5 equivalents) distilled and purified immediately before the procedure were added to the reaction mixture at the same temperature, and the mixture was heated and refluxed for 24 hours under a nitrogen stream. The reaction solution was cooled to 95°C to 105°C, the reaction was checked by HPLC, and the raw material was found to remain. Copper powder (259 g, 1 equivalent) was added thereto, and the mixture was heated and refluxed for 20 hours. The reaction solution was cooled to 95°C to 105°C, the reaction was checked by HPLC, and the raw material was found to remain. Copper powder (259 g, 1 equivalent) was added thereto again, and the mixture was heated and refluxed for 20 hours. At this time, the conversion rate of the reaction was 98.7%.

The reaction solution was cooled to room temperature and then filtered through a short column packed with silica gel (100 to 200 mesh, 6.5 kg).

The short column was washed with ethyl acetate (44 L) and methanol (22 L). The collected filtrate and washings were concentrated under reduced pressure, and the residue was dissolved in ethyl acetate (22 L). To the obtained solution, 6% aqueous ammonia (11 L) was added, and the mixture was stirred for 20 minutes. The organic layer was separated, and then the aqueous layer was extracted three times with ethyl acetate (11 L). The collected organic layer was dried over anhydrous sodium sulfate (1.0 kg), then insolubles were filtered off, and the filtrate was concentrated under reduced pressure. The residue was dissolved in a mixed solvent (11 L) of ethyl acetate and heptane (1 : 4) and cooled to 0 to 10°C. A 4 N hydrochloric acid/ethyl acetate solution (2.2 L, 2.2 equivalents) was added to the obtained solution with stirring, and then the mixture was stirred for 1 hour. The obtained solid was collected by filtration and washed with a mixed solvent (11 L) of ethyl acetate and heptane (1 : 4) to obtain a dihydrochloride of the title compound.

¹H-NMR (400 MHz, CD₃OD) δ7.51 - 7.62 (m, 5H), 7.40 (d, 1H, J = 12 Hz), 7.25 - 7.37 (m, 2H), 7.14 (d, 1H, J = 8.4 Hz), 6.78 (d, 1H, J = 12 Hz), 6.71 (d, 1H, J = 12 Hz), 4.09 - 4.47 (m, 5H), 2.79 - 3.88 (m, 11H), 1.15 - 2.10 (m, 21H), 0.70 - 0.90 (m, 2H), 0.47 - 0.69 (m, 2H).

The dihydrochloride obtained above was transferred to a 100 L reaction vessel, and ethyl acetate (11 L), water (11 L), and concentrated aqueous ammonia (about 4.5 L) were added thereto to adjust the pH of the aqueous layer from 10 to 11.

The organic layer was separated, and then the aqueous layer was extracted three times with ethyl acetate (11 L). The collected organic layer was concentrated under reduced pressure and filtered through a short column packed with silica gel (100 to 200 mesh, 5.5 kg). The short column was washed with ethyl acetate (44 L) and methanol (22 L). The collected filtrate and washings were concentrated under reduced pressure, the obtained residue was dissolved in 2-propanol (16.5 L), and the obtained solution was slowly added dropwise to vigorously stirred water (66 L) at room temperature. The resulting solid was collected by filtration and washed with water (500 mL).

The obtained solid was dried at 25°C for 72 hours under reduced pressure to obtain a free form of the title compound (1.85 kg, 75.9%, purity: 90.5%) as a white solid.

¹H-NMR (400 MHz, CD₃OD) δ7.20 - 7.30 (m, 7H), 7.03 (d, 1H, J = 8.4 Hz), 6.88 (d, 1H, J = 8.4 Hz), 6.59 - 6.63 (m, 2H), 3.58 - 3.64 (m, 4H), 2.93 - 3.32 (m, 7H), 2.65 - 2.75 (m, 2H), 2.45 - 2.49 (m, 1H), 2.26 - 2.35 (m, 2H), 1.98 - 2.05 (m, 1H), 1.69 - 1.78 (m, 2H), 1.44 - 1.57 (m, 1H), 1.22 - 1.34 (m, 1H), 1.01 - 1.16 (m, 2H), 0.72 - 0.82 (m, 2H), 0.40 - 0.51 (m, 2H), 0.08 - 0.18 (m, 2H).

### (Production method in which catalytic amount of cuprous iodide is used)

Pyridine (175 mL) was added to a 250 mL reaction vessel at room temperature, (1S,3aR,5aS,6R,11bS,11cS)-3-benzyl-14-(cyclopropylmethyl)-10-methoxy-2,3,3a,4,5,6,7,11c-octahydro-1H-6,11b-(epiminoethano)-1,5a-methanonaphtho[1,2-e]indole-11-ol dihydrochloride (10.87 g, 20.0 mmol, 1 equivalent) and potassium phosphate (19.1 g, 90 mmol, 4.5 equivalents) were added thereto at room temperature under a nitrogen atmosphere, and then the mixture was stirred at 95 to 105°C for 60 minutes. Then, copper iodide (0.95 g, 5.0 mmol, 0.25 equivalents) and 1-bromo-4-(tert-butyl)benzene (17.1 g, 80 mmol, 4.0 equivalents) were added thereto at the same temperature, then the reaction temperature was raised to 110 to 120°C, and the mixture was stirred for 4 hours under a nitrogen atmosphere. HPLC analysis was performed, and the conversion rate was found to be 54%. Subsequently, stirring was continued for 14 hours, the disappearance of the raw material was confirmed by HPLC analysis, and then the reaction solution was cooled to room temperature. The reaction solution was filtered through a Seitz filter, insolubles were filtered off, and then the filter was washed with ethyl acetate (260 mL). The combined filtrate and washings were concentrated under reduced pressure to obtain a residue (24.7 g). Ethyl acetate (300 mL) and 6% aqueous ammonia (160 mL) were added to the residue to adjust the pH to 10, and the mixture was stirred for 20 minutes. The aqueous layer was separated and then extracted with ethyl acetate (300 mL), the combined ethyl acetate layers were washed with water (400 mL) and filtered through a Pall-Carbon filter, and the filtrate was concentrated under reduced pressure to obtain a residue (19.8 g). The residue was dissolved in ethyl acetate (80 mL), then the obtained solution was cooled to 0 to 10°C, and a 1 M hydrogen chloride/ethyl acetate solution was slowly added. Then, heptane (33 mL) was added thereto, and the mixture was stirred at 0 to 10°C for 30 minutes. The obtained suspension was filtered and washed with a mixed solvent of ethyl acetate and heptane (1 : 2, 100 mL x 2) to obtain a dihydrochloride of the title compound (12.0 g, 88.5%) as a light brown amorphous powder (94.7%: purity).

The NMR spectrum was consistent with the NMR spectrum of that produced using copper powder.

The dihydrochloride (6.72 g, 0.01 mol, 1 equivalent) obtained above was added to a reaction vessel, and dissolved in water (220 mL) for 20 minutes while stirring. To the obtained solution, 25% aqueous ammonia (2.7 g, 0.04 mol, 4 equivalents) was added over 30 minutes with stirring, and then the mixture was stirred at room temperature for 30 minutes. The obtained suspension was stirred at 0 to 5°C for 2 hours, and the solid was collected by filtration, washed with water (200 mL x 2), and then dried at 40°C under reduced pressure to obtain a free form of the title compound (5.2 g, 87%) as a light brown amorphous solid (purity 90.3%).

The NMR spectrum was consistent with the NMR spectrum of that produced using copper powder.

### (Example 1-1)

Production of (1S,3aR,5aS,6R,11bS,11cS)-14-(cyclopropylmethyl)-10-methoxy-11-phenoxy-2,3,3a,4,5,6,7,11c-octahydro-1H-6,11b-(epiminoethano)-1,5a-methanonaphtho[1,2-e]indole

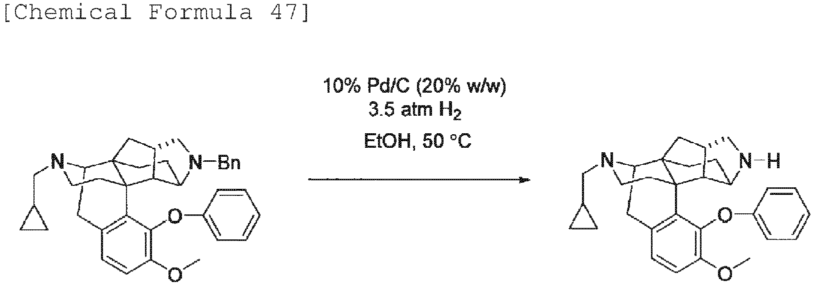

### (Production by debenzylation by hydrogenation)

A free form (22.9 g, 1.0 equivalent) adjusted according to a conventional method from (lS,3aR,5aS,6R,llbS,llcS)-3-benzyl-14-(cyclopropylmethyl)-10-methoxy-11-phenoxy-2,3,3a,4,5,6,7,11c-octahydro-1H-6,11b-(epiminoethano)-1,5a-methanonaphtho[1,2-e]indole dihydrochloride was dissolved in ethanol (230 mL), 10% palladium-carbon (6.87 g) was added thereto, and the mixture was stirred under a hydrogen atmosphere (50 psi) at 40 °C. After 32 hours, the raw material was found to remain by HPLC analysis, thus the reaction solution was filtered, 10% palladium-carbon (6.97 g) was added to the filtrate, and the mixture was further stirred at 40°C for 56 hours under a hydrogen atmosphere (50 psi) (conversion rate: 92%). The raw material was found to remain by HPLC analysis, and thus stirring was further continued for 3 days. The reaction solution was filtered off and washed with ethanol (100 mL), and then the filtrate was concentrated under reduced pressure to obtain the title compound (19.5 g, 85%).

¹H-NMR (400 MHz, CD₃OD) δ6.75 - 7.21 (m, 7H), 2.83 - 3.91 (m, 13H), 0.60 - 2.51 (m, 12H), 0.35 - 0.58 (m, 2H), 0.02 - 0.10 (m, 2H).

### (Example 1-2)

Production of (1S,3aR,5aS,6R,11bS,11cS)-14-(cyclopropylmethyl)-10-methoxy-11-phenoxy-2,3,3a,4,5,6,7,11c-octahydro-1H-6,11b-(epiminoethano)-1,5a-methanonaphtho[1,2-e]indole

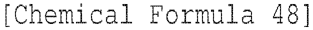

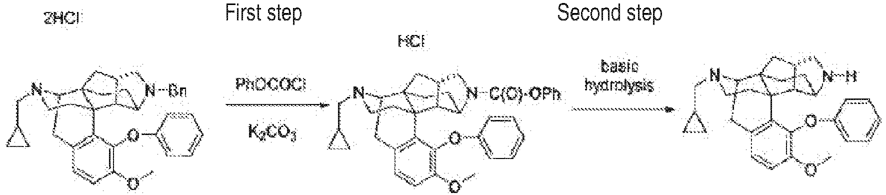

### (Production by debenzylation via phenyl carbamate)

First step: production of phenyl(1S,3aR,5aS,6R,11bS,11cS)-14-(cyclopropylmethyl)-10-methoxy-11-phenoxy-1,2,3a,4,5,6,7,11c-octahydro-3H-6,11b-(epiminoethano)-1,5a-methanonaphtho[1,2-e]indole-3-carboxylate hydrochloride

THF (50 mL, 10 v/w) was added to a 100 mL reaction vessel, (1S,3aR,5aS,6R,11bS,11cS)-3-benzyl-14-(cyclopropylmethyl)-10-methoxy-11-phenoxy-2,3,3a,4,5,6,7,11c-octahydro-1H-6,11b-(epiminoethano)-1,5a-methanonaphtho[1,2-e]indole dihydrochloride (5.0 g, 8.1 mmol, 1 equivalent) was suspended at room temperature, and then water (1.5 mL, 0.3 v/w) and potassium carbonate (5.6 g, 40.4 mmol, 5 equivalents) were added. The reaction mixture changed from suspension to clear solution. The reaction mixture was stirred under heating and refluxing conditions, then phenyl chloroformate (2.54 g, 16.2 mmol, 2 equivalents) was added thereto, and the mixture was stirred at 60°C for 2 hours. The disappearance of the raw material was confirmed by HPLC analysis.

The reaction solution was cooled to room temperature, then ethyl acetate (50 mL, 10 v/w) and water (25 mL, 5.0 v/w) were added thereto, and the mixture was stirred at room temperature for 30 minutes.

The two layers were separated, and the organic layer was washed twice with water (50 mL, 10 v/w) and concentrated under reduced pressure. The residue was dissolved in a mixed solvent of ethyl acetate and heptane (1 : 8, 100 mL, 20 v/w), the obtained solution was cooled to 0 to 10°C, a 4 N hydrogen chloride/ethyl acetate solution (8.1 mL, 32.4 mmol, 4 equivalents) was slowly added thereto with stirring, and the mixture was stirred for 1 hour.

The resulting solid was collected by filtration and washed with a mixed solvent of ethyl acetate and heptane (1 : 8, 25 mL, 5 v/w). The obtained solid was added to a 100 mL reaction vessel and suspended in MTBE (25 mL, 5 v/w), and the mixture was stirred for 1 hour under superheated reflux conditions and then cooled to 0 to 10°C. A solid was collected by filtration, washed with MTBE (10 mL, 2.0 v/w), and then dried at 45 to 50°C under reduced pressure for 3 hours to obtain the title compound (4.5 g, 93.9%) as a light yellow powder (purity: 91%).

¹H-NMR (400 MHz, CD₃OD) δ6.75 - 7.21 (m, 12H), 4.35 - 4.63 (m, 1H), 4.20 (d, 1H, J = 6.4 Hz), 2.71 - 3.95 (m, 10H), 0.71 - 1.88 (m, 14H), 0.43 - 0.60 (m, 2H).

### Second step: production of

(1S,3aR,5aS,6R,11bS,11cS)-14-(cyclopropylmethyl)-10-methoxy-11-phenoxy-2,3,3a,4,5,6,7,11c-octahydro-1H-6,11b-(epiminoethano)-1,5a-methanonaphtho[1,2-e]indole tert-Butanol (86 mL, 20 v/w) was added to a 250 mL reaction vessel, phenyl(1S,3aR,5aS,6R,11bS,11cS)-14-(cyclopropylmethyl)-10-methoxy-11-phenoxy-1,2,3a,4,5,6,7,11c-octahydro-3H-6,11b-(epiminoethano)-1,5a-methanonaphtho[1,2-e]indole-3-carboxylate hydrochloride (4.3 g, 7.0 mmol, 1 equivalent) obtained above was added thereto, and the solvent was distilled off at 50 to 55°C under reduced pressure with stirring. tert-Butanol (43 mL, 10 v/w) was added again, and the solvent was distilled off in the same manner. The above operation was repeated once more and the resulting product was cooled to room temperature. The obtained residue was suspended in tert-butanol (43 mL, 10 v/w), potassium hydroxide (3.9 g, 70.2 mmol, 10 equivalents) was added thereto, and the reaction solution was stirred for 2 hours under heating and refluxing conditions. HPLC analysis was performed, and the raw material was found to be completely consumed. The reaction solution was cooled to room temperature, then MTBE (86 mL, 20.0 v/w) was added thereto, and the mixture was washed with water (86 mL, 20.0 v/w). The aqueous layer was extracted twice with MTBE (43 mL, 10.0 v/w), and the collected organic layer was concentrated under reduced pressure. The residue was suspended in MTBE (86 mL, 20.0 v/w), and water (86 mL, 20.0 v/w) and 12 N hydrochloric acid were added to adjust the pH to 3. The aqueous layer was separated and washed twice with MTBE (43 mL, 10.0 v/w).

The aqueous layer was transferred to a 250 mL reaction vessel and stirred under reduced pressure to distill off MTBE. The remaining aqueous layer was heated to 50°C and stirred for 1 hour, then aqueous ammonia was added thereto at the same temperature to adjust the pH to 9 to 10, and the mixture was further stirred for 1 hour and then cooled to room temperature to obtain a suspension. The solid was collected by filtration, washed with water (4 mL, 1.0 v/w), and dried at 45 to 50°C under reduced pressure for 3 hours to obtain the title compound (2.4 g, 74.9%) as a light yellow powder (purity 99.7%).

The NMR spectrum was consistent with the NMR spectrum of that produced by the method by debenzylation by hydrogenation.

### (Example 2-1)

Production of (1S,3aR,5aS,6R,11bS,11cS)-11-(4-(tert-butyl)phenoxy)-14-(cyclopropylmethyl)-10-methoxy-2,3,3a,4,5,6,7,11c-octahydro-1H-6,11b-(epiminoethano)-1,5a-methanonaphtho[1,2-e]indole

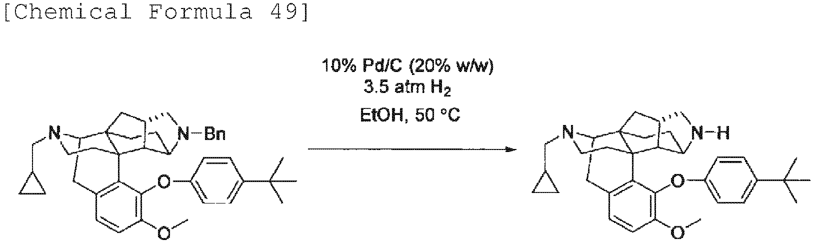

### (Production by debenzylation by hydrogenation)

Ethanol (4.5 L) was added to a 10 L autoclave, and (1S,3aR,5aS,6R,11bS,11cS)-3-benzyl-11-(4-(tert-butyl)phenoxy)-14-(cyclopropylmethyl)-10-methoxy-2,3,3a,4,5,6,7,11c-octahydro-1H-6,11b-(epiminoethano)-1,5a-methanonaphtho[1,2-e]indole (435 g, 0.73 mol) produced in Reference Example 2 and 10% palladium-carbon (87 g) were added thereto at room temperature. The inside of the reaction vessel was replaced with nitrogen three times, then replaced with hydrogen three times, the internal pressure was set to 0.3 to 0.35 MPa, and the mixture was stirred at 50°C one day. At this time, the raw material still remained, thus the reaction temperature was reduced to 25°C to 30°C, and the inside of the reaction vessel was replaced with nitrogen three times. The palladium-carbon was filtered off and then washed with ethanol (150 mL).

The collected filtrate and washings were transferred to a 10 L autoclave, 10% palladium-carbon (87 g) was added thereto, the inside of the reaction vessel was replaced with nitrogen three times, and then replaced with hydrogen three times, the internal pressure was set to 0.3 to 0.35 MPa, and the mixture was stirred at 50°C for 72 hours. After the inside of the reaction vessel was replaced with nitrogen three times, the palladium-carbon was filtered off and washed with THF (1.0 L). The collected filtrate and washings were concentrated under reduced pressure to obtain the title compound (330 g, 89.2%) as a brown syrup (89.8%: purity).

¹H-NMR (400 MHz, CD₃OD) δ7.26 - 7.30 (m, 2H), 7.10 (d, 1H, J = 8.8 Hz), 6.97 (d, 1H, J = 8.4 Hz), 6.64 - 6.68 (m, 2H), 3.84 - 3.88 (m, 1H), 2.88 - 3.44 (m, 8H), 2.51 - 2.55 (m, 2H), 2.30 - 2.40 (m, 2H), 2.06 - 2.12 (m, 1H), 1.71 - 1.87 (m, 2H), 1.46 - 1.54 (m, 1H), 1.02 - 1.24 (m, 1H), 0.75 - 0.80 (m, 1H), 0.49 - 0.51 (m, 2H), 0.01 - 0.02 (m, 2H).

### (Example 2-2)

Production of (1S,3aR,5aS,6R,11bS,11cS)-11-(4-(tert-butyl)phenoxy)-14-(cyclopropylmethyl)-10-methoxy-2,3,3a,4,5,6,7,11c-octahydro-1H-6,11b-(epiminoethano)-1,5a-methanonaphtho[1,2-e]indole

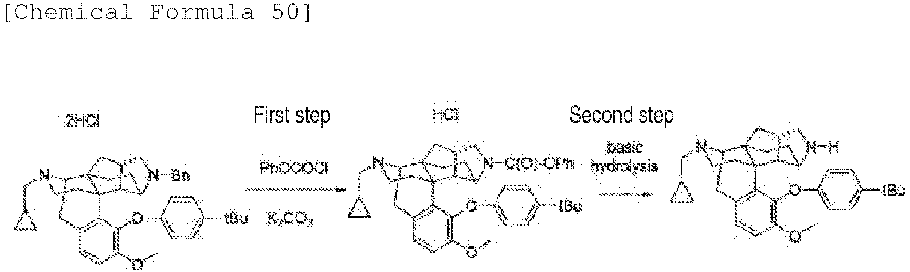

### (Production by debenzylation via phenyl carbamate)

First step: production of phenyl(1S,3aR,5aS,6R,11bS,11cS)-11-(4-(tert-butyl)phenoxy)-14-(cyclopropylmethyl)-10-methoxy-1,2,3a,4,5,6,7,11c-octahydro-3H-6,11b-(epiminoethano)-1,5a-methanonaphtho[1,2-e]indole-3-carboxylate hydrochloride
THF (10.0 L, 10.0 v/w) was added to a 20 L reaction vessel, and (1S,3aR,5aS,6R,11bS,11cS)-3-benzyl-11-(4-(tert-butyl)phenoxy)-14-(cyclopropylmethyl)-10-methoxy-2,3,3a,4,5,6,7,11c-octahydro-1H-6,11b-(epiminoethano)-1,5a-methanonaphtho[1,2-e]indole dihydrochloride was further added thereto and suspended at room temperature. Water (300 mL, 0.3 v/w) and potassium carbonate (1022.0 g, 7.39 mol, 5 equivalents) were added to the obtained mixture at room temperature, and the mixture was stirred under heating and refluxing conditions. Phenyl chloroformate (464.0 g, 2.96 mol, 2 equivalents) was added to the reaction solution at 60°C over 10 minutes. After completion of the dropwise addition, the mixture was stirred for 2 hours. The conversion rate was found to be 99.8% by HPLC analysis.

The reaction solution was cooled to room temperature, then ethyl acetate (10.0 L, 10 v/w) and water (5.0 L, 5 v/w) were added thereto, and the mixture was stirred at room temperature for 30 minutes. The two layers were separated, and the organic layer was washed twice with water (10.0 L, 10 v/w). The organic layer was concentrated under reduced pressure, the residue was dissolved in a mixed solvent of ethyl acetate and heptane (1 : 8, 20.0 L, 20 v/w), the obtained solution was cooled to 0 to 10°C, a 4 N hydrogen chloride/ethyl acetate solution (1.48 L, 4.0 equivalents) was slowly added, and the mixture was stirred for 1 hour. The obtained suspension was filtered, and the solid was filtered off, and then washed with a mixed solvent of ethyl acetate and heptane (1 : 8, 5.0 L, 5 v/w) to obtain a yellow solid.

The obtained solid was transferred to a 10 L reaction solution, and suspended in MTBE (5.0 L, 5 v/w), and the mixture was stirred for 1 hour under heating and refluxing conditions. The reaction solution was cooled to 0 to 10°C, and the solid was collected by filtration, then washed with MTBE (1.0 L, 1 v/w), and then dried at 45 to 50°C for 12 hours under reduced pressure to obtain the title compound (947.0 g, 95.6%) as a yellow solid (purity: 92.9%).

¹H-NMR (400 MHz, CD₃OD) δ7.03 - 7.38 (m, 11H), 4.19 - 4.47 (m, 2H), 2.72 - 3.97 (m, 10H), 1.90 - 2.02 (m, 2H), 1.40 - 1.80 (m, 4H), 1.21 - 1.39 (m, 11H), 1.00 - 1.20 (m, 2H), 0.70 - 1.00 (m, 4H), 0.45 - 0.59 (m, 2H).

### Second step: production of

(1S,3aR,5aS,6R,11bS,11cS)-11-(4-(tert-butyl)phenoxy)-14-(cyclopropylmethyl)-10-methoxy-2,3,3a,4,5,6,7,11c-octahydro-1H-6,11b-(epiminoethano)-1,5a-methanonaphtho[1,2-e]indole
tert-Butanol (13.0 L, 10 v/w) was added to a 20 L reaction vessel, and phenyl(1S,3aR,5aS,6R,11bS,11cS)-11-(4-(tert-butyl)phenoxy)-14-(cyclopropylmethyl)-10-methoxy-1,2,3a,4,5,6,7,11c-octahydro-3H-6,11b-(epiminoethano)-1,5a-methanonaphtho[1,2-e]indole-3-carboxylate hydrochloride (1.3 kg, 1.94 mol, 1 equivalent) was further added thereto and suspended. The obtained mixture was concentrated with stirring at 50 to 55°C until the amount of the solvent became 5.0 to 6.5 L. tert-Butanol (6.5 L, 5 v/w) was added to the residue at room temperature, and the solvent was distilled off again. This operation was repeated again.

The reaction solution was cooled to room temperature, then tert-butanol (6.5 L, 5 v/w) and potassium hydroxide (1.09 kg, 19.4 mol, 10 equivalents) were added thereto, and the mixture was stirred for 2 hours under heating and refluxing conditions. The disappearance of the raw material was confirmed by HPLC analysis, and the reaction solution was cooled to room temperature.

MTBE (13.0 L, 10.0 v/w) was added to the reaction vessel and washed with water (13.0 L, 10.0 v/w). The aqueous layer was extracted twice with MTBE (6.5 L, 5 v/w), and the collected organic layer was concentrated under reduced pressure. The residue was dissolved in MTBE (13.0 L, 10.0 v/w), water (26.0 L, 20.0 v/w) was added thereto, and then 36% hydrochloric acid (450 mL) was added thereto to adjust the pH to 3 to 4.

The two layers were separated, and the aqueous layer was washed twice with MTBE (6.5 L, 5 v/w). The aqueous layer was concentrated under reduced pressure, and MTBE was distilled off.

The remaining aqueous layer was heated to 50°C, and 25% aqueous ammonia (380.0 g) was added to adjust the pH to 9 to 10. The obtained suspension was filtered, and the solid was filtered off, then washed with water (13 L, 10 v/w), and then dried at 55 to 60°C under reduced pressure for 6 days to obtain the title compound (895.0 g, 90.0%) as a light yellow solid (purity: 94.3%).

The NMR spectrum was consistent with the NMR spectrum of that produced by debenzylation by hydrogenation.

### (Experimental results)

Hereinafter, the yield and purity of the compound described in Reference Example 1-1 of Patent Literature 11 ([Chemical Formula 6] of [0024]) obtained by subjecting the diphenyl ether derivatives (starting materials) obtained in Reference Examples 1 and 2 and Examples 1-1 to 2-2 to Benkeser reaction conditions in which lithium metal is used are shown in Table 1.

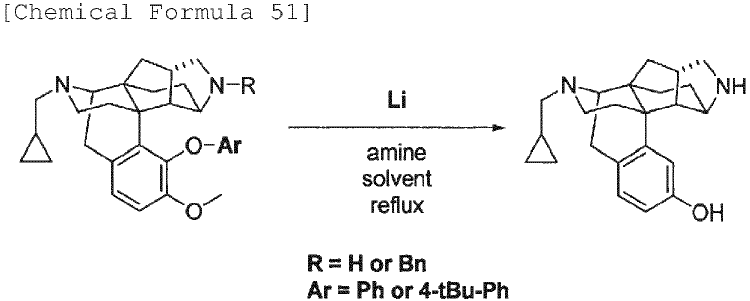

**[Table 1]**

| Example | R | Ar | Starting material | Amine | Solvent | Yield amount/ Yield | Purity |
|---|---|---|---|---|---|---|---|
| 3 | Bn | Ph | 1. 0 g | Ethylenediamine | THF | 0.3 g 47% | 82% |
| 4 | Bn | Ph | 1.0 g | Ethylenediamine | none | 0.2 g 31% | 95.4% |
| 5 | Bn | 4-tBuPh | 1.0 g | Ethylenediamine | none | 0.18 g 31% | 96.7% |
| 6 | H | Ph | 5.0 g | Ethylenediamine | THF | 3.6 g 94% | 79.3% |
| 7 | H | Ph | 2.2 g | Ethylenediamine | none | 0.86 g 51 % | 99.1% |
| 8 | H | 4-tBuPh | 1440 g | Ethylenediamine | THF | 460 g 46.6% | 94.0% |
| 9 | H | 4-tBuPh | 50 g | Ethylenediamine | none | 23.8 g 69.5% | 98.4% |

### (Example 3)

Production of (1S,3aR,5aS,6R,11bR,11cS)-14-(cyclopropylmethyl)-2,3,3a,4,5,6,7,11c-octahydro-1H-6,11b-(epiminoethano)-1,5a-methanonaphtho[1,2-e]indole-10-ol 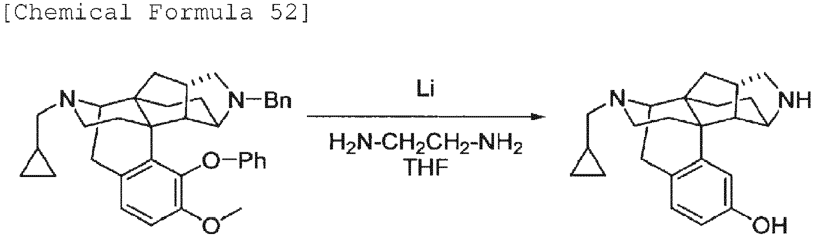 THF (5 mL) and lithium (70 mg, 5 equivalents) were added to a 50 mL 3-necked flask and cooled to 0°C, then a THF (5 mL) solution of ethylenediamine (5 mL, 41 equivalents) and (1S,3aR,5aS,6R,11bS,11cS)-3-benzyl-14-(cyclopropylmethyl)-10-methoxy-11-phenoxy-2,3,3a,4,5,6,7,11c-octahydro-1H-6,11b-(epiminoethano)-1,5a-methanonaphtho[1,2-e]indole (1 g, 1 equivalent) was added at the same temperature, and the mixture was stirred for 1 hour. The reaction solution was heated to room temperature and stirred for 2 hours, then lithium (70 mg, 5 equivalents) was added thereto, and the mixture was stirred for 1 hour at the same temperature. The raw material was found to be completely consumed by HPLC analysis.

Ethanol (5 mL) and water (5 mL) were added to the reaction solution, and then the pH of the reaction solution was adjusted to 7 with 1 N hydrochloric acid. The reaction solution was concentrated under reduced pressure, ethanol was distilled off, and then a residue containing water was extracted twice with ethyl acetate (20 mL). The pH of the aqueous layer was adjusted to 11 to 12 using a 1 M aqueous sodium hydroxide solution, then the aqueous layer was extracted three times with ethyl acetate (20 mL), and the organic layer was concentrated under reduced pressure to obtain the title compound (0.3 g, 47%). Purity: 82%

### The structure of the obtained

(1S,3aR,5aS,6R,11bR,11cS)-14-(cyclopropylmethyl)-2,3,3a,4,5,6,7,11c-octahydro-1H-6,11b-(epiminoethano)-1,5a-methanonaphtho[1,2-e]indole-10-ol was confirmed by the retention time consistent with that of the compound described in Reference Example 1-1 of Patent Literature 11 ([Chemical Formula 6] of [0024]) in HPLC analysis.

### (Example 4)

Production of (1S,3aR,5aS,6R,11bR,11cS)-14-(cyclopropylmethyl)-2,3,3a,4,5,6,7,11c-octahydro-1H-6,11b-(epiminoethano)-1,5a-methanonaphtho[1,2-e]indole-10-ol 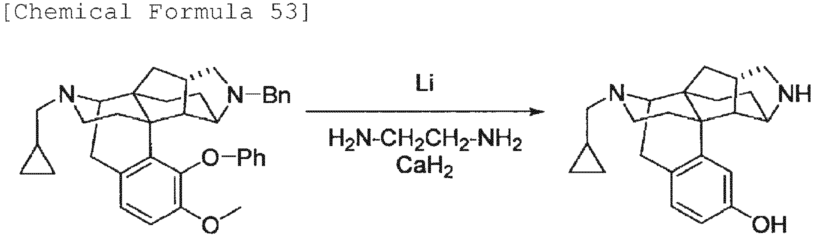 Ethylenediamine (40 mL, 353 equivalents) and calcium hydride (2.0 g, 5.0%) were added to a reaction vessel, heated and refluxed for 1 hour, and then cooled to 60°C. To the obtained suspension, (1S,3aR,5aS,6R,11bS,11cS)-3-benzyl-14-(cyclopropylmethyl)-10-methoxy-11-phenoxy-2,3,3a,4,5,6,7,11c-octahydro-1H-6,11b-(epiminoethano)-1,5a-methanonaphtho[1,2-e]indole (1.0 g, 1 equivalent) was added at 60°C, and the mixture was stirred at 60°C for 15 minutes. Lithium chips (512 mg, 40 equivalents) were added to the reaction solution, and the mixture was stirred at 60°C for 30 minutes. During this time, the internal temperature of the reaction solution increased to 80°C. After the disappearance of the raw material was confirmed by HPLC, the reaction solution was cooled to 0°C to 10°C. THF (20 mL) and methanol (3 mL) were added to the reaction vessel at the same temperature, and then 6 N hydrochloric acid (10 mL) was added thereto at the same temperature to adjust the pH of the reaction solution to 10 to 11. The obtained suspension was filtered, and insolubles were filtered off and washed with THF (20 mL). The filtrate was extracted three times with a mixed solvent of isopropyl acetate and THF (2 : 1) (20 mL). The collected organic layer was washed with 15% brine (40 mL), and then concentrated under reduced pressure. Isopropyl acetate (6 mL) and water (6 mL) were added to the residue to dissolve the residue. The mixture of the obtained two layers was stirred at room temperature for one day to obtain a suspension. The resulting solid was washed with isopropyl acetate (2.0 mL) to obtain the title compound (0.2 g, 31%, purity: 95.4%) as a yellow solid. The structure of the obtained (1S,3aR,5aS,6R,11bR,11cS)-14-(cyclopropylmethyl)-2,3,3a,4,5,6,7,11c-octahydro-1H-6,11b-(epiminoethano)-1,5a-methanonaphtho[1,2-e]indole-10-ol was confirmed by the retention time consistent with that of the compound described in Reference Example 1-1 of Patent Literature 11 ([Chemical Formula 6] of [0024]) in HPLC analysis.

### (Example 5)

Production of (1S,3aR,5aS,6R,11bR,11cS)-14-(cyclopropylmethyl)-2,3,3a,4,5,6,7,11c-octahydro-1H-6,11b-(epiminoethano)-1,5a-methanonaphtho[1,2-e]indole-10-ol 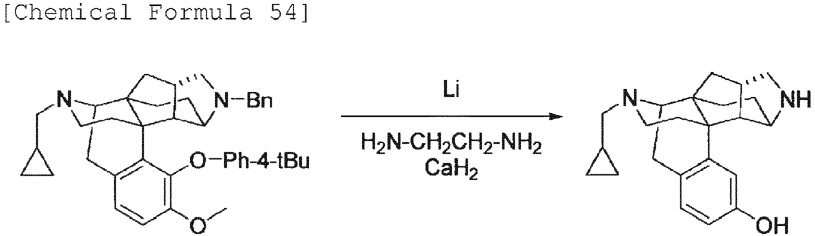 Ethylenediamine (40 mL, 353 equivalents) and calcium hydride (2.0 g, 5.0%) were added to a reaction vessel, heated and refluxed for 1 hour, and then cooled to 60°C.

To the obtained suspension, (1S,3aR,5aS,6R,11bS,11cS)-3-benzyl-11-(4-(tert-butyl)phenoxy)-14-(cyclopropylmethyl)-10-methoxy-2,3,3a,4,5,6,7,11c-octahydro-1H-6,11b-(epiminoethano)-1,5a-methanonaphtho[1,2-e]indole (1.0 g, 1 equivalent) was added at 60°C, and the mixture was stirred at 60°C for 15 minutes. Lithium chips (465 mg, 40 equivalents) were added to the reaction solution, and the mixture was stirred at 60°C for 30 minutes. During this time, the internal temperature of the reaction solution increased to 80°C. After the disappearance of the raw material was confirmed by HPLC, the reaction solution was cooled to 0°C to 10°C. THF (20 mL) and methanol (3 mL) were added to the reaction vessel at the same temperature, and then 6 N hydrochloric acid (10 mL) was added thereto at the same temperature to adjust the pH of the reaction solution to 10 to 11. The obtained suspension was filtered, and insolubles were filtered off and washed with THF (20 mL). The filtrate was extracted three times with a mixed solvent of isopropyl acetate and THF (2 : 1) (20 mL). The collected organic layer was washed with 15% brine (40 mL), and then concentrated under reduced pressure. Isopropyl acetate (60 mL) and water (60 mL) were added to the residue to dissolve the residue. The mixture of the obtained two layers was stirred at room temperature for one day to obtain a suspension. The resulting solid was washed with isopropyl acetate (2.0 mL) to obtain the title compound (0.18 g, 31%, purity: 96.7%) as a yellow solid.

### The structure of the obtained

(1S,3aR,5aS,6R,11bR,11cS)-14-(cyclopropylmethyl)-2,3,3a,4,5,6,7,11c-octahydro-1H-6,11b-(epiminoethano)-1,5a-methanonaphtho[1,2-e]indole-10-ol was confirmed by the retention time consistent with that of the compound described in Reference Example 1-1 of Patent Literature 11 ([Chemical Formula 6] of [0024]) in HPLC analysis.

### (Example 6)

Production of (1S,3aR,5aS,6R,11bR,11cS)-14-(cyclopropylmethyl)-2,3,3a,4,5,6,7,11c-octahydro-1H-6,11b-(epiminoethano)-1,5a-methanonaphtho[1,2-e]indole-10-ol 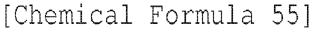 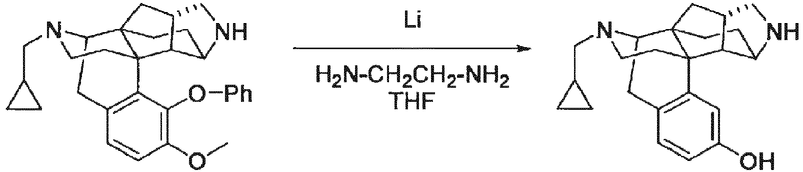 THF (50 mL), lithium chips (760 mg, 10 equivalents), and ethylenediamine (12.5 mL, 17 equivalents) were added to a 500 mL three-necked flask, and the mixture was heated and refluxed for 30 minutes. A THF (10 mL) solution of (1S,3aR,5aS,6R,11bS,11cS)-14-(cyclopropylmethyl)-10-methoxy-11-phenoxy-2,3,3a,4,5,6,7,11c-octahydro-1H-6,11b-(epiminoethano)-1,5a-methanonaphtho[1,2-e]indole (5 g, 1 equivalent) was added to the obtained solution, and the mixture was heated and refluxed for 2.5 hours. The disappearance of the raw material was confirmed by HPLC analysis.

The reaction solution was cooled to 0 to 10°C, and ethanol (20 mL) and water (20 mL) were added to stop the reaction. The reaction solution was concentrated under reduced pressure, ethanol was distilled off, and then concentrated hydrochloric acid was added to a residue containing water to adjust the pH to 1 to 2. The aqueous layer was washed twice with ethyl acetate (50 mL), then a 4 N aqueous sodium hydroxide solution was added to the aqueous layer to adjust the pH to 11 to 12, and then the mixture was extracted three times with ethyl acetate (50 mL). The collected organic layer was dried over anhydrous sodium sulfate, then insolubles were filtered off and dried under reduced pressure to obtain the title compound (3.6 gg, 94%, purity: 79.3%) as a yellow solid.

### The structure of the obtained

(1S,3aR,5aS,6R,11bR,11cS)-14-(cyclopropylmethyl)-2,3,3a,4,5,6,7,11c-octahydro-1H-6,11b-(epiminoethano)-1,5a-methanonaphtho[1,2-e]indole-10-ol was confirmed by the retention time consistent with that of the compound described in Reference Example 1-1 of Patent Literature 11 ([Chemical Formula 6] of [0024]) in HPLC analysis.

### (Example 7)

Production of (1S,3aR,5aS,6R,11bR,11cS)-14-(cyclopropylmethyl)-2,3,3a,4,5,6,7,11c-octahydro-1H-6,11b-(epiminoethano)-1,5a-methanonaphtho[1,2-e]indole-10-ol 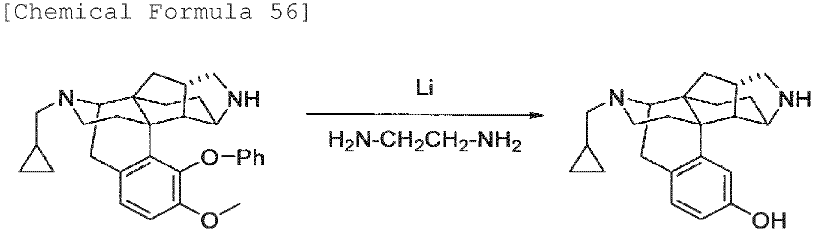 Ethylenediamine (33 mL, 15 v/w) and calcium hydride (1.65 g, 5 wt%) were added to a 250 mL reaction vessel at room temperature, and the mixture was stirred at 100 to 120°C for 30 minutes. The reaction solution was cooled to 60 to 70°C, then (1S,3aR,5aS,6R,11bS,11cS)-14-(cyclopropylmethyl)-10-methoxy-11-phenoxy-2,3,3a,4,5,6,7,11c-octahydro-1H-6,11b-(epiminoethano)-1,5a-methanonaphtho[1,2-e]indole (2.2 g, 4.82 mmol, 1 equivalent) was added thereto at the same temperature, and the mixture was stirred for 30 minutes. Lithium (101 mg, 14.4 mmol, 3 equivalents) was added thereto, the mixture was stirred for 10 minutes, then lithium (101 mg, 14.4 mmol, 3 equivalents) was added again, the mixture was stirred for 10 minutes, then lithium (136 mg, 19.4 mmol, 4 equivalents) was further added thereto, and the mixture was stirred for 60 minutes. HPLC analysis was performed, and disappearance of the raw material was confirmed. Thus, the reaction solution was cooled to 0 to 5°C, THF (33 mL, 15 v/w) and methanol (7 mL, 3 v/w) were added at the same temperature, and then 6 N hydrochloric acid (22 mL, 10 v/w) was added to adjust the pH to 10 to 11. The aqueous layer was separated, and then extracted three times with a mixed solvent of isopropyl acetate and THF (44 mL, 20 v/w). 4 N hydrochloric acid was added to the collected organic layer to adjust the pH to 1 to 2. The aqueous layer was separated, and then the organic layer was extracted twice with 4 N hydrochloric acid (22 mL, 10 v/w).

Isopropyl acetate (22 mL, 10 v/w) was added to the collected aqueous layer, and the pH was adjusted to 10 with a 6 N aqueous sodium hydroxide solution. The mixture of the obtained two layers was stirred at room temperature for 1 hour and then stirred at 0 to 5°C for 2 hours. The obtained suspension was filtered, and the collected solid was washed with a mixture of isopropyl acetate and water (4 mL, 2 v/w) and dried to obtain the title compound (0.86 g, 51%) as a light yellow solid. Purity: 99.1%

### The structure of the obtained

(1S,3aR,5aS,6R,11bR,11cS)-14-(cyclopropylmethyl)-2,3,3a,4,5,6,7,11c-octahydro-1H-6,11b-(epiminoethano)-1,5a-methanonaphtho[1,2-e]indole-10-ol was confirmed by the retention time consistent with that of the compound described in Reference Example 1-1 of Patent Literature 11 ([Chemical Formula 6] of [0024]) in HPLC analysis.

### (Example 8)

Production of (1S,3aR,5aS,6R,11bR,11cS)-14-(cyclopropylmethyl)-2,3,3a,4,5,6,7,11c-octahydro-1H-6,11b-(epiminoethano)-1,5a-methanonaphtho[1,2-e]indole-10-ol

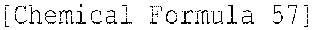

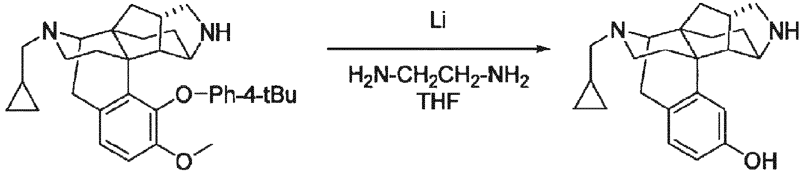

In a 100 L reaction vessel, (1S,3aR,5aS,6R,11bS,11cS)-11-(4-(tert-butyl)phenoxy)-14-(cyclopropylmethyl)-10-methoxy-2,3,3a,4,5,6,7,11c-octahydro-1H-6,11b-(epiminoethano)-1,5a-methanonaphtho[1,2-e]indole (1440 g, 1 equivalent) was dissolved in THF (21.6 L) at room temperature, lithium chips (157 g, 8 equivalent) were added thereto, and the mixture was stirred at 40 to 50°C for 20 minutes. Ethylenediamine (2529 g, 15 equivalents) was added to the reaction mixture at the same temperature, and the mixture was stirred at 60 to 70°C for 120 minutes. The disappearance of the raw material was confirmed by HPLC, and the reaction vessel was cooled to 0 to 10°C. Ethanol (3.6 L) was added to the reaction mixture, the mixture was stirred for 30 minutes, then water (3.6 L) was added thereto, and the mixture was further stirred for 30 minutes. The obtained suspension was filtered, solids were washed twice with THF (3 L), and then the combined filtrate and washings were transferred to 50 L of a reaction solution. Water (15 L) was added to the reaction vessel, the organic layer and the aqueous layer were separated, and then the aqueous layer was extracted twice with a mixed solvent (7.5 L) of isopropyl acetate and THF (2 : 1). 4 N hydrochloric acid (9 L) was added to the collected organic layer. At this time, the pH of the aqueous layer was 1 to 2. The organic layer and aqueous layer were separated, and the organic layer was extracted with 4 N hydrochloric acid (3 L). The collected aqueous layer was washed with isopropyl acetate (6 L), and then isopropyl acetate (6 L) and a 6 N aqueous sodium hydroxide solution (9 L) were added to the aqueous layer. At this time, the pH of the aqueous layer was 10. The mixture of the obtained two layers was stirred at room temperature for one day to obtain a suspension. The resulting solid was collected by filtration, washed twice with isopropyl acetate (3 L), and then dried at 40 to 45°C for 4 hours under reduced pressure to obtain the title compound (460 g, 47%, purity: 94%) as a light yellow solid.

### The structure of the obtained

(1S,3aR,5aS,6R,11bR,11cS)-14-(cyclopropylmethyl)-2,3,3a,4,5,6,7,11c-octahydro-1H-6,11b-(epiminoethano)-1,5a-methanonaphtho[1,2-e]indole-10-ol was confirmed by the retention time consistent with that of the compound described in Reference Example 1-1 of Patent Literature 11 ([Chemical Formula 6] of [0024]) in HPLC analysis.

### (Example 9)

Production of (1S,3aR,5aS,6R,11bR,11cS)-14-(cyclopropylmethyl)-2,3,3a,4,5,6,7,11c-octahydro-1H-6,11b-(epiminoethano)-1,5a-methanonaphtho[1,2-e]indole-10-ol 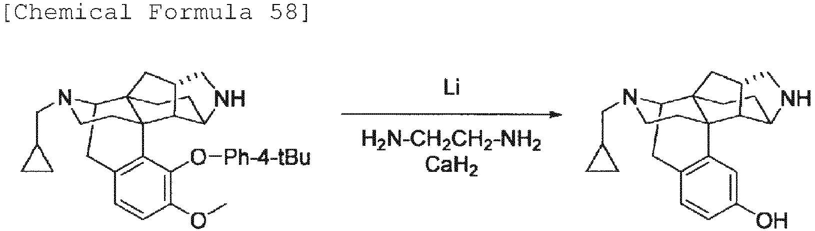 Ethylenediamine (675.0 g, 115.1 equivalents) and calcium hydride (37.5 g, 5%) were added to a 5 L reaction vessel at room temperature. The obtained mixture was heated and refluxed for 1 hour. The reaction solution was cooled to 60 to 65°C, then (1S,3aR,5aS,6R,11bS,11cS)-11-(4-(tert-butyl)phenoxy)-14-(cyclopropylmethyl)-10-methoxy-2,3,3a,4,5,6,7,11c-octahydro-1H-6,11b-(epiminoethano)-1,5a-methanonaphtho[1,2-e]indole (50 g, 1 equivalent) was added thereto at the same temperature, and the mixture was stirred for 15 minutes. Lithium chips (684 mg, 1 equivalent) were added to the reaction mixture, and the mixture was stirred for 20 minutes. A small amount of foaming was observed. When the foaming subsided, lithium chips (1.368 g, 2 equivalents) were added to the reaction mixture again, and the mixture was stirred for 20 minutes. Similarly, after the foaming subsided, lithium chips (1.368 g, 2 equivalents) were added to the reaction mixture, and the mixture was stirred for 20 minutes. Similarly, after the foaming subsided, lithium chips (1.368 g, 2 equivalents) were added to the reaction mixture, and the mixture was stirred for 20 minutes. Similarly, after the foaming subsided, lithium chip (2.052 g, 3 equivalents) were added to the reaction mixture, and the mixture was stirred for 1 hour. The internal temperature of the reaction solution spontaneously increased from 60°C to 70°C due to exotherm. The disappearance of the starting material was confirmed by HPLC, and thus the reaction solution was cooled to 0 to 10°C. THF (750 mL), methanol (150 mL), and 6 N hydrochloric acid (300 mL) were added thereto at the same temperature, and the pH of the reaction solution was adjusted to 10 to 11 to obtain a suspension. The resulting suspension was filtered, and the residue was washed with THF (300 mL). The aqueous layer and organic layer of the filtrate were separated, the aqueous layer was extracted three times with a mixed solvent of isopropyl acetate and THF (2 : 1) (300 mL), and the collected organic layer was concentrated under reduced pressure. The residue was dissolved in toluene (100 mL), the mixture was stirred at 50 to 60°C for 1 hour, and then cooled to 0 to 10°C to obtain a suspension. The resulting solid was collected by filtration, washed with toluene (5.0 mL), and then dried to obtain the title compound (23.8 g, 69.5%, purity: 98.4%) as a light yellow solid.

### The structure of the obtained

(1S,3aR,5aS,6R,11bR,11cS)-14-(cyclopropylmethyl)-2,3,3a,4,5,6,7,11c-octahydro-1H-6,11b-(epiminoethano)-1,5a-methanonaphtho[1,2-e]indole-10-ol was confirmed by the retention time consistent with that of the compound described in Reference Example 1-1 of Patent Literature 11 ([Chemical Formula 6] of [0024]) in HPLC analysis.

### Industrial Applicability

According to the present invention, it is possible to provide a method for converting a biphenyl ether form having an ortho-methoxy group into a monophenol derivative in one step. The method provided by the present invention can be applied to a compound having a morphinan skeleton.

## Claims

1. A method for producing a phenol derivative represented by General Formula (B) below: 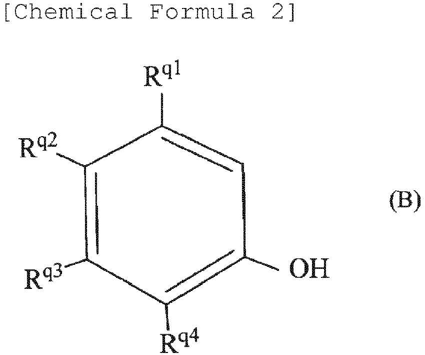
wherein R^{q1}, R^{q2}, R^{q3}, and R^{q4} are same or different, and represent a hydrogen atom or any substituent, or
adjacent two of R^{q1}, R^{q2}, R^{q3}, and R^{q4} optionally form a ring; or
a salt, a tautomer, a stereoisomer, or an isotope of the phenol derivative, comprising the step of:
allowing metal lithium and liquid ammonia or a primary amine to act on a compound represented by General Formula (A) below: [Chemical Formula 1] 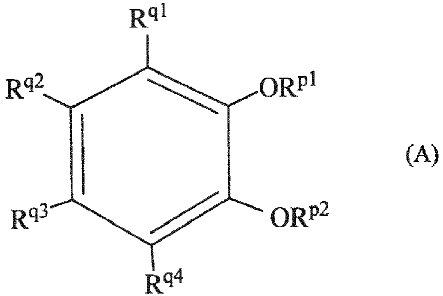
wherein R^{p1} represents a C₆₋₁₀ aryl group optionally having a substituent,
R^{p2} represents a lower alkyl group, an aralkyl group optionally having a substituent, an alkenyl group optionally having a substituent, or a silyl protecting group, and
R^{q1}, R^{q2}, R^{q3}, and R^{q4} have a meaning same as above; or
a tautomer, a stereoisomer, or an isotope of the compound.

2. The method according to claim 1, wherein R^{q3} and R^{q4} are a hydrogen atom, and R^{q1} and R^{q2} together form a ring.

3. The method according to claim 1 or 2, wherein R^{p1} is a phenyl group.

4. The method according to any one of claims 1 to 3, wherein R^{p2} is a methyl group.

5. A method for producing of a morphinan derivative represented by General Formula (II) below: 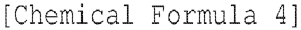 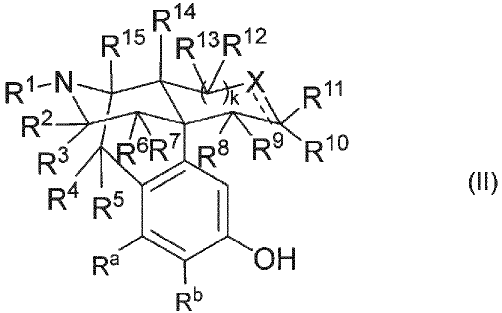
wherein R¹ to R¹⁵, R^{a}, and R^{b} are same or different, and represent a hydrogen atom or any substituent
wherein any two groups selected from R⁸, R¹⁰, and R¹⁴ are optionally bonded to each other to form an alkylene chain, the alkylene chain is optionally substituted with a substituent, a carbon atom that constitutes the alkylene chain is optionally replaced with a heteroatom, the alkylene chain optionally further has a double bond or an amide bond in middle, and rest other than the selected two groups is optionally bonded to the alkylene chain,
X indicates a carbon atom optionally having a substituent, a nitrogen atom optionally having a substituent, or an oxygen atom,
k represents 1 or 2, and
a double line consisting of a solid line and a broken line represents a single bond or a double bond
wherein
when the double line consisting of a solid line and a broken line is a double bond,
X is N or CR⁰¹ wherein R⁰¹ is a hydrogen atom or any substituent, and in this case R¹¹ is absent,
when the double line consisting of a solid line and a broken line is a single bond,
X is O, NR⁰², CR⁰³R⁰⁴, C=CR^{c0}R^{d0}, or C=NCₓ
wherein R⁰², R⁰³, and R⁰⁴ are same or different, and are a hydrogen atom or any substituent,
R^{c0} and R^{d0} are same or different, and are a hydrogen atom, a C₁₋₁₀ alkyl group optionally having a substituent, a C₃₋₁₀ alkenyl group optionally having a substituent, an aryl group optionally having a substituent, or a heteroaryl group optionally having a substituent,
Cₓ represents a C₁₋₁₀ alkyl group optionally having a substituent, and
R¹⁰ and R¹¹, together with a carbon atom to which R¹⁰ and R¹¹ are bonded, represent C=CR^{c}R^{d} (R^{c} and R^{d} are same or different, and indicate a hydrogen atom, a C₁₋₁₀ alkyl group optionally having a substituent, a C₃₋₁₀ alkenyl group optionally having a substituent, an aryl group optionally having a substituent, or a heteroaryl group optionally having a substituent) or C=NC_{y} (C_{y} indicates a C₁₋₁₀ alkyl group optionally having a substituent), or optionally form a cyclic ketal optionally having a substituent, and further, when X is NR⁰², together with a carbon atom to which R¹⁰ and R¹¹ are bonded, are optionally a carbonyl group or a thiocarbonyl group,
the R⁰¹ and R¹⁰ are optionally bonded to each other to form an unsaturated hydrocarbon ring optionally having a substituent, an unsaturated heterocycle optionally having a substituent, or a lactam ring optionally having a substituent, and the unsaturated hydrocarbon ring, the unsaturated heterocycle, and the lactam ring are optionally further fused with a saturated hydrocarbon ring optionally having a substituent, a saturated heterocycle optionally having a substituent, an unsaturated hydrocarbon ring optionally having a substituent, or an unsaturated heterocycle optionally having a substituent,
the R⁰² or R⁰³ and R¹⁰ are optionally bonded to each other to form a saturated hydrocarbon ring optionally having a substituent, a saturated heterocycle optionally having a substituent, an unsaturated hydrocarbon ring optionally having a substituent, an unsaturated heterocycle optionally having a substituent, or a lactam ring optionally having a substituent, and the saturated hydrocarbon ring, the saturated heterocycle, the unsaturated hydrocarbon ring, the unsaturated heterocycle, and the lactam ring are optionally further fused with a saturated hydrocarbon ring optionally having a substituent, a saturated heterocycle optionally having a substituent, an unsaturated hydrocarbon ring optionally having a substituent, or an unsaturated heterocycle optionally having a substituent,
the R^{c0} and R^{c}, together with C=C to which R^{c0} and R^{c} are bonded, optionally form an unsaturated hydrocarbon ring or an unsaturated heterocycle,
the R^{c0} and C_{y}, together with C=C and C=N to which R^{c0} and C_{y} are bonded, optionally form an unsaturated heterocycle,
the Cₓ and C_{y}, together with C=N to which Cₓ and C_{y} are bonded, optionally form an unsaturated heterocycle; or
a salt, a tautomer, a stereoisomer, or an isotope of the morphinan derivative, comprising the step of:
allowing metal lithium and an amine to act on a morphinan derivative represented by General Formula (I) below: 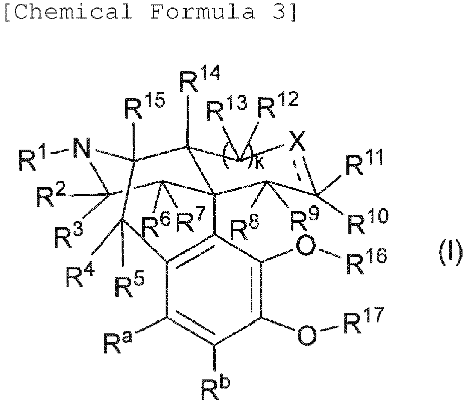
wherein R¹ to R¹⁵, R^{a}, and R^{b} have a meaning same as above,
R¹⁶ represents a C₆₋₁₀ aryl group optionally having a substituent or a heteroaryl group optionally having a substituent,
R¹⁷ represents a C₁₋₁₀ alkyl group, an aralkyl group optionally having a substituent, an alkenyl group optionally having a substituent, or a silyl protecting group,
X, a double line consisting of a solid line and a broken line, and k have a meaning same as above; or
a tautomer, a stereoisomer, or an isotope of the morphinan derivative in presence or absence of an organic solvent.

6. The method according to claim 5, wherein k is 1, X is CH₂, and the double line consisting of a solid line and a broken line is a single bond.

7. The method according to claim 5 or 6, wherein R¹ is a hydrogen atom, a methyl group, a cyclopropylmethyl group, a cyclobutylmethyl group, a benzyl group, or an allyl group.

8. The method according to any one of claims 5 to 7, wherein R¹ is a hydrogen atom.

9. The method according to any one of claims 5 to 8, wherein R² to R⁷, R⁹, R¹¹ to R¹³, R¹⁵, R^{a}, and R^{b} are same or different, and are a hydrogen atom, a methyl group, a cyclopropylmethyl group, a cyclobutylmethyl group, a benzyl group, or an allyl group.

10. The method according to any one of claims 5 to 9, wherein R⁸, R¹⁰, and R¹⁴ together form two rings containing a carbon atom to which R⁸, R¹⁰, and R¹⁴ are bonded.

11. The method according to any one of claims 5 to 10, wherein R¹⁶ is a C₆₋₁₀ aryl group optionally having a substituent.

12. The method according to any one of claims 5 to 10, wherein R¹⁶ is a phenyl group.

13. The method according to any one of claims 5 to 12, wherein R¹⁷ is a methyl group.

14. The method according to any one of claims 5 to 13, wherein the amine is liquid ammonia, a primary amine, or a secondary amine.

15. The method according to any one of claims 5 to 13, wherein the amine is a primary amine.

16. The method according to any one of claims 5 to 13, wherein the amine is a primary amine represented by General Formula (III) below: 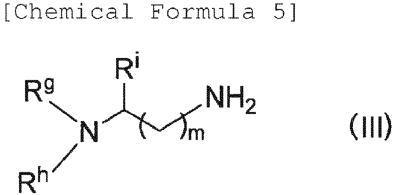 wherein R^{g}, R^{h}, and Rⁱ are same or different, and represent a hydrogen atom or a C₁₋₆ alkyl group optionally having a substituent or any two of R^{g}, R^{h}, and Rⁱ together optionally form a ring, and m represents an integer of 1 to 5.

17. The method according to any one of claims 5 to 16, wherein metal lithium is used in an amount of 2 to 20 equivalents relative to one equivalent of one functional group to be reduced in the morphinan derivative represented by the General Formula (I).

18. The method according to any one of claims 5 to 17, wherein the organic solvent is an aromatic hydrocarbon solvent, an alcohol solvent, or an ether solvent.

19. The method according to any one of claims 5 to 17, wherein the organic solvent is an ether solvent.

20. The method according to any one of claims 5 to 17, wherein metal lithium and an amine are allowed to act on the morphinan derivative represented by the General Formula (I) in absence of an organic solvent.

21. The method according to any one of claims 5 to 20, wherein a reaction temperature is -10°C to 120°C.

22. The method according to any one of claims 5 to 20, wherein a reaction temperature is -5°C to 105°C.

23. The method according to any one of claims 5 to 22, wherein a metal hydride is present in a reaction system.

24. The method according to claim 22, wherein the metal hydride is selected from lithium hydride, sodium hydride, potassium hydride, calcium hydride, lithium borohydride, sodium borohydride, diisobutylaluminum hydride, and lithium aluminum hydride.

25. A method for producing a morphinan derivative represented by General Formula (V) below: 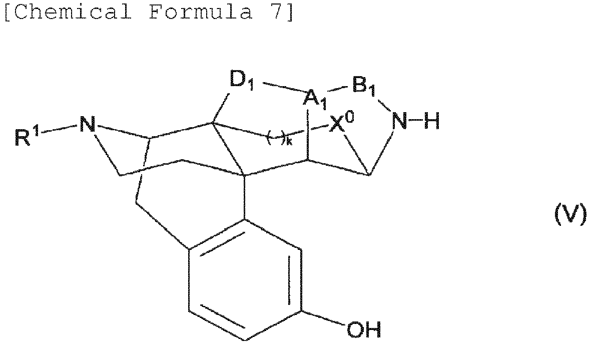
wherein R¹ represents a hydrogen atom or any substituent,
k represents 1 or 2,
X⁰ represents a carbon atom optionally having a substituent, a nitrogen atom optionally having a substituent, or an oxygen atom,
A₁ indicates CH or N,
B₁ indicates an alkylene chain having 1 to 3 carbon atoms and optionally having a substituent, and the alkylene chain, together with NR⁰, optionally forms an amide bond, and
D₁ indicates CH₂, NR⁰⁵, O, or S wherein R⁰⁵ represents a hydrogen atom or any substituent; or
a salt, a tautomer, a stereoisomer, or an isotope of the morphinan derivative, comprising the step of:
allowing metal lithium and an amine to act on a morphinan derivative represented by General Formula (IV) below: 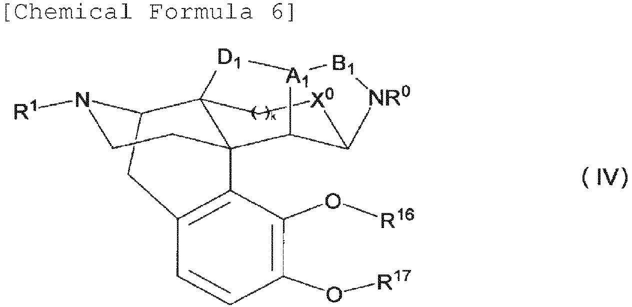
wherein R¹ have a meaning same as above,
R¹⁶ represents a C₆₋₁₀ aryl group optionally having a substituent or a heteroaryl group optionally having a substituent,
R¹⁷ represents a C₁₋₁₀ alkyl group, an aralkyl group optionally having a substituent, an alkenyl group optionally having a substituent, or a silyl protecting group,
k, X⁰, A₁, B₁, and D₁ have a meaning same as above, and
R⁰ represents a hydrogen atom, an aralkyl group optionally having a substituent, or a heteroarylalkyl group optionally having a substituent; or
a tautomer, a stereoisomer, or an isotope of the morphinan derivative in presence or absence of an organic solvent.

26. The method according to claim 25, wherein k is 1, and X⁰ is CH₂.

27. The method according to claim 25 or 26, wherein B₁ and D₁ are CH₂, and A₁ is CH.

28. The method according to any one of claims 25 to 27, wherein R¹ is a hydrogen atom, a methyl group, a cyclopropylmethyl group, a cyclobutylmethyl group, a benzyl group, or an allyl group.

29. The method according to any one of claims 25 to 28, wherein R¹ is a hydrogen atom.

30. The method according to any one of claims 25 to 29, wherein R¹⁶ is a C₆₋₁₀ aryl group optionally having a substituent.

31. The method according to any one of claims 25 to 29, wherein R¹⁶ is a phenyl group.

32. The method according to any one of claims 25 to 31, wherein R¹⁷ is a methyl group.

33. The method according to any one of claims 25 to 32, wherein R⁰ is a hydrogen atom or a benzyl group.

34. The method according to any one of claims 25 to 33, wherein the amine is liquid ammonia, a primary amine, or a secondary amine.

35. The method according to any one of claims 25 to 33, wherein the amine is a primary amine.

36. The method according to any one of claims 25 to 33, wherein the amine is a primary amine represented by General Formula (III) below: 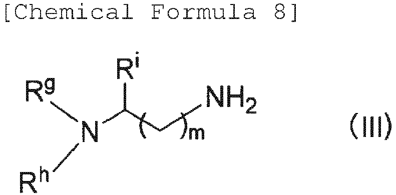 wherein R^{g}, R^{h}, and Rⁱ are same or different, and represent a hydrogen atom or a C₁₋₆ alkyl group optionally having a substituent or any two of R^{g}, R^{h}, and Rⁱ together optionally form a ring, and m represents an integer of 1 to 5.

37. The method according to any one of claims 25 to 36, wherein metal lithium is used in an amount of 2 to 20 equivalents relative to one functional group to be reduced in the morphinan derivative represented by the General Formula (IV).

38. The method according to any one of claims 25 to 37, wherein the organic solvent is an aromatic hydrocarbon solvent, a lower alcohol solvent, or an ether solvent.

39. The method according to any one of claims 25 to 37, wherein the organic solvent is an ether solvent.

40. The method according to any one of claims 25 to 37, wherein metal lithium and an amine are allowed to act on the morphinan derivative represented by the General Formula (IV) in absence of an organic solvent.

41. The method according to any one of claims 25 to 40, wherein a reaction temperature is -10°C to 120°C.

42. The method according to any one of claims 25 to 40, wherein a reaction temperature is -5°C to 105°C.

43. The method according to any one of claims 25 to 42, wherein a metal hydride is present in a reaction system.

44. The method according to claim 43, wherein the metal hydride is selected from lithium hydride, sodium hydride, potassium hydride, calcium hydride, lithium borohydride, sodium borohydride, diisobutylaluminum hydride, and lithium aluminum hydride.

45. A morphinan derivative represented by General Formula (VI) below: 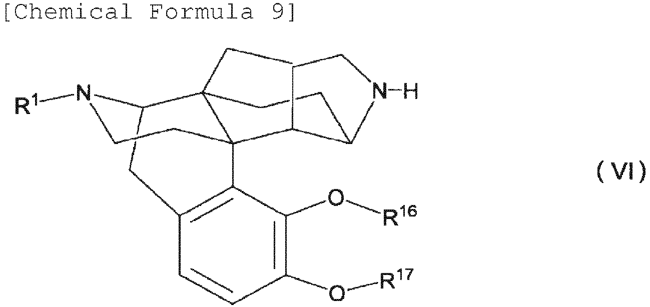
wherein R¹ represents a hydrogen atom or any substituent,
R¹⁶ represents a C₆₋₁₀ aryl group optionally having a substituent or a heteroaryl group optionally having a substituent, and
R¹⁷ represents a C₁₋₁₀ alkyl group, an aralkyl group optionally having a substituent, an alkenyl group optionally having a substituent, or a silyl protecting group; or
a tautomer, a stereoisomer, or an isotope of the morphinan derivative.

46. The morphinan derivative according to claim 45; or a tautomer, a stereoisomer, or an isotope of the morphinan derivative, wherein R¹ is a hydrogen atom, a methyl group, a cyclopropylmethyl group, a cyclobutylmethyl group, a benzyl group, or an allyl group.

47. The morphinan derivative according to claim 45 or 46; or a tautomer, a stereoisomer, or an isotope of the morphinan derivative, wherein R¹ is a hydrogen atom.

48. The morphinan derivative according to any one of claims 45 to 47; or a tautomer, a stereoisomer, or an isotope of the morphinan derivative, wherein R¹⁶ is a C₆₋₁₀ aryl group optionally having a substituent.

49. The morphinan derivative according to any one of claims 45 to 47; or a tautomer, a stereoisomer, or an isotope of the morphinan derivative, wherein R¹⁶ is a phenyl group.

50. The morphinan derivative according to any one of claims 46 to 49; or a tautomer, a stereoisomer, or an isotope of the morphinan derivative, wherein R¹⁷ is a methyl group.
